(19) Europäisches Patentamt
European Patent Office
Office européen des brevets

(11) Veröffentlichungsnummer: **0 298 404 B1**

(12) **EUROPÄISCHE PATENTSCHRIFT**

(45) Veröffentlichungstag der Patentschrift: **12.01.94**

(51) Int. Cl.[5]: **C07C 403/00**, C07C 317/00

(21) Anmeldenummer: **88110639.7**

(22) Anmeldetag: **04.07.88**

# (54) Sulfonylpolyene.

(30) Priorität: **09.07.87 CH 2615/87**
**09.05.88 CH 1749/88**

(43) Veröffentlichungstag der Anmeldung:
**11.01.89 Patentblatt 89/02**

(45) Bekanntmachung des Hinweises auf die Patenterteilung:
**12.01.94 Patentblatt 94/02**

(84) Benannte Vertragsstaaten:
**AT BE CH DE FR GB IT LI NL**

(56) Entgegenhaltungen:
**FR-A- 2 138 212**
**FR-A- 2 171 497**

**J. AM. CHEM. SOC., Band 106, 1984, S. 3670-3672; T. MANDAI et al: "Novel synthesis of acetylenes and polyenes via desulfonylation reaction"**

**TETRAHEDRON LETTERS, Band 23, Nr. 32, 1982, Seiten 3265-3266, Pergamon Press, Ltd, GB; J. BREMNER et al: "Synthèse à l'aide de sulfones (XXIV). Synthèse stéréosélective d'oléfines par hydrogénolyse des vinylsulfones"**

(73) Patentinhaber: **F. HOFFMANN-LA ROCHE AG**
**Postfach 3255**
**CH-4002 Basel(CH)**

(72) Erfinder: **Bernhard, Kurt, Dr.**
**Bündtenstrasse 11**
**CH-4411 Lupsingen(CH)**
Erfinder: **Jäggli, Stephan**
**Bündtenstrasse 21**
**CH-4416 Bubendorf(CH)**
Erfinder: **Kreienbühl, Paul, Dr.**
**Chrischonaweg 69**
**CH-4125 Riehen(CH)**
Erfinder: **Schwieter, Ulrich, Dr.**
**Im Pfeiffengarten 8**
**CH-4153 Reinach(CH)**

(74) Vertreter: **Cottong, Norbert A. et al**
**Grenzacherstrasse 124**
**Postfach 3255**
**CH-4002 Basel (CH)**

**EP 0 298 404 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von Carotinoiden und Carotinoid-Zwischenprodukten sowie neue Sulfonylpolyene in diesem Verfahren und deren Herstellung.

Es ist grundsätzlich bekannt, dass Sulfone mit Aldehyden und Ketonen β-Hydroxysulfone bilden können und dass die Sulfonylgruppe nach Dehydratisierung des β-Hydroxysulfons zum Vinylsulfon auf reduktivem Weg eliminiert werden kann. Die bekannten Reduktionsmethoden sind jedoch im allgemeinen nur für einfache Moleküle geeignet und oft nicht stereospezifisch. Beispielsweise ist die Reduktion mit Natriumdithionit und Natriumhydrogencarbonat für Polyene meist nicht oder nur schlecht geeignet. Anderseits sind die β-Hydroxysulfone wenig stabil und im Fall der vorliegenden Polyensysteme nicht herstellbar, da offenbar ein reversibler Zerfall in die Edukte eintritt.

Die bisher bakannten Anwendungen von Sulfonen bei der Herstellung von Carotinoiden betreffen daher Reaktionen mit Halogeniden, Estern etc. zu Sulfonen, welche die Sulfonylgruppe an einer gesättigten C-C-Bindung aufweisen und aus denen die Sulfonylgruppe durch Umsetzung mit Base eliminiert werden kann.

Die vorliegende Erfindung betrifft nun ein Verfahren zur Herstellung der Verbindungen der allgemeinen Formel

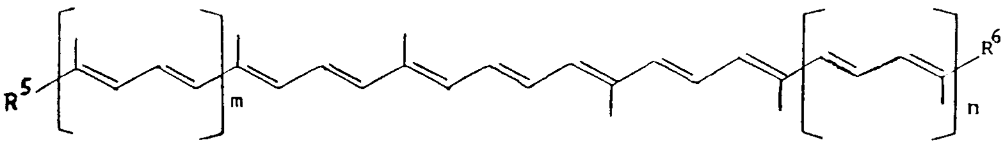

III

worin m die Zahl 1 und $R^5$ 4-Methyl-3-pentenyl bezeichnet, oder m die Zahl 0 und $R^5$ eine gegbenenfalls acetalisierte Formylgruppe, eine gegebenenfalls veresterte Carboxygruppe oder eine Gruppe der allgemeinen Formel

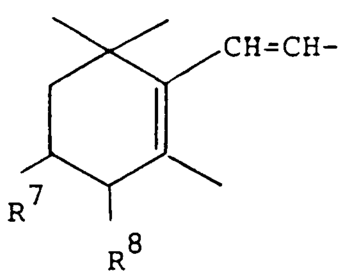

IV

bezeichnet; n die Zahl 1 und $R^6$ 4-Methyl-3-pentenyl bezeichnet, oder n die Zahl 0 und $R^6$ eine gegbenenfalls acetalisierte Formylgruppe, eine gegbenenfalls veresterte Carboxygruppe oder eine Gruppe der Formel IV bezeichnet; und $R^7$ und $R^8$ unabhängig voneinander Wasserstoff, eine gegebenenfalls geschützte Hydroxygruppe oder eine gegebenenfalls geschützte Oxogruppe darstellen,

dadurch gekennzeichent, dass man eine Verbindung der allgemeinen Formel

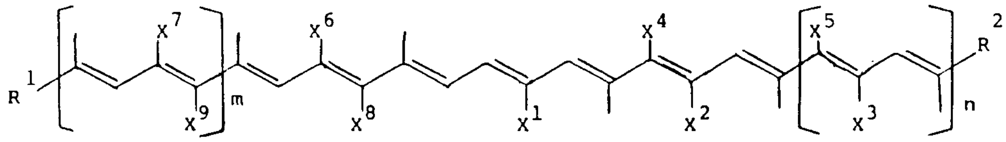

I

worin m die Zahl 1 und $R^1$ 4-Methyl-3-pentenyl bezeichnet, oder m die Zahl 0 und $R^1$ eine acetalisierte Formylgruppe, eine gegebenenfalls veresterte Carboxygruppe oder eine Gruppe der allgemeinen Formel

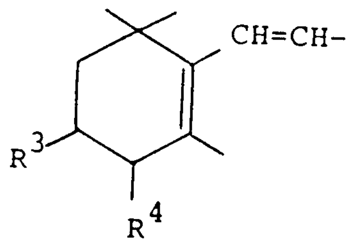

II

bezeichent; n die Zahl 1 und $R^2$ 4-Methyl-3-pentenyl bezeichnet, oder n die Zahl 0 und $R^2$ eine acetalisierte Formylgruppe, eine gegebenenfalls veresterte Carboxygruppe oder eine Gruppe der Formel II bezeichnet; $R^3$ und $R^4$ unabhängig voneinander Wasserstoff, eine gegebenenfalls geschüuzte Hydroxygruppe oder eine geschützte Oxogruppe darstellen; eine der vorhandenen Gruppen $X^1$, $X^2$, $X^3$, $X^4$ und $X^5$ eine Sulfonylrest bedeutet und die übrigen Wasserstoff bedeuten; und $X^5$, $X^7$, $X^5$ und $X^5$ Wasserstoff bedeuten oder, wenn $R^1$ und $R^2$ gleiche Bedeutungen haben, auch $X^5$ die Bedeutung von $X^2$, $X^7$ die Bedeutung von $X^3$, $X^5$ die Bedeutung von $X^4$ und $X^5$ die Bedeutung von $X^5$ hat,
mit Dithionit in einem wässrig-organischen Lösungsmittelgemisch in Gegenwart von Ammoniak oder einem aliphatischen Amin reduziert und dass man, gewünschten falls, vorhandene Schutzgruppen abspaltet.

Der Ausdruck "acetalisierte Formylgruppe" umfasst im Rahmen der vorliegenden erfindung übliche acetalisierte Aldehydgruppen, insbesondere Gruppen der Formel $-CH(OR^{13})_2$, worin jeder der Reste $R^{13}$ $C_1$-$C_5$-Alkyl oder beide Reste zusammen $C_2$-$C_6$-Alkylen bedeuten, wie Dimethoxymethyl oder 1,3-Dioxolan-2-yl.

Der Ausdruck "veresterte Carboxygruppe" umfasst übliche veresterte Carboxyreste, insbesondere ($C_1$-$C_5$-Alkoxy)carbonyl, wie Methoxycarbonyl, Aethoxycarbonyl und dergleichen.

Der Ausdruck "geschützte Hydroxygruppe" umfasst übliche geschützte Hydroxygruppen, insbesondere Acyloxygruppen und verätherte Hydroxygruppen.

Der Ausdruck "Acyloxygruppe" umfasst übliche Carbonsäureesterreste, insbesondere Alkanoyloxy und Aroyloxy mit bis zu 8 Kohlenstoffatomen, wie Formyloxy, Acetoxy, Propionyloxy oder Benzoyloxy.

Der Ausdruck "verätherte Hydroxygruppe" umfasst übliche Aetherschutzgruppen, insbesondere $C_1$-$C_5$-Alkoxy, wie Methoxy oder Aethoxy, und Gruppen der Formel $-O-CR^{14}R^{15}-OR^{16}$, worin $R^{14}$ und $R^{15}$ Wasserstoff oder $C_1$-$C_5$-Alkyl und $R^{16}$ $C_1$-$C_5$-Alkyl bedeuten oder $R^{15}$ und $R^{16}$ zusammen auch $C_2$-$C_6$-Alkylen bedeuten, wie 1-Methoxy-1-methyläthoxy oder Tetrahydropyranyloxy.

Der Ausdruck "geschützte Oxogruppe" umfasst übliche geschützte Oxogruppen. Bevorzugt sind die acetalisierten Oxogruppen, insbesondere diejenigen, worin der Ausdruck geschützte Oxogruppe für zwei $C_1$-$C_5$-Alkoxygruppen (z.B. für 2 Methoxygruppen) oder für eine $C_2$-$C_6$-Alkylendioxygruppe (z.B. Aethylendioxy oder 2,3-Butylendioxy) steht. Ferner kann eine Oxogruppe auch als Enoläther geschützt sein. Die Methode ist vor allem im Fall von $\alpha$-Hydroxyketonen bevorzugt, wobei die Verütherung des Endiols vorzugsweise auch durch Dimerisierung oder durch Bildung eines cyclischen Acetals (z.B. mit Acton zum Actonid) erfolgen kann.

Der Ausdruck "Sulfonylrest" umfasst im Rahmen der vorliegenden Erfindung übliche in Julia-Reaktionen verwendbare Sulfonylreste. Vorzugsweise steht der Ausdruck für substituiertes oder unsubstituiertes Phenylsulfonyl, insbesondere für Reste der Formel $-SO_2-R^{17}$, worin $R^{17}$ unsubstituiertes oder mit Halogen, Phenyl, Phenoxy, $C_1$-$C_5$-Alkyl und/oder $C_1$-$C_5$-Alkoxy substituiertes Phenyl beduetet, wie Phenylsulfonyl, p-Chlorphenylsulfonyl, 4-Biphenylylsulfonyl, p-Phenoxyphenylsulfonyl, p-Tolylsulfonyl, p-Methoxyphenylsulfonyl und dergleichen.

Der Ausdruck "Halogen" umfasst Fluor, Chlor, Brom und Jod.

Der Ausdruck "Alkyl" umfasst geradkettige und verzweigte Gruppen mit vorzugsweise 1-5 Kohlenstoffatomen, wie Methyl, Aethyl, Propyl, Isopropyl, t-Butyl und dergleichen. Der Ausdruck "Alkoxy" umfasst Alkyloxygruppen, worin "Alkyl" die genannten bedeutungen hat. Der Ausdruck "Alkylen" umfasst geradkettige und verzweigte Gruppen mit vorzugsweise 2-6 Kohlenstoffatomen, z.B. 2,3-Butylen oder insbesondere Polymethylen, wie Aethylen, Trimethylen, Tetramethylen, Pentamethylen, Hexamethylen und dergleichen.

Der Ausdruck "aliphatisches Amin" umfasst im Rahmen der vorliegenden Erfindung Amine und Hydroxylamine, worin das mit der Aminogruppe direkt verknüpfte Kohlenstoffatom gesättigt ist, bzw. die mit der Aminogruppe direkt verknüpften Kohlenstoffatome gesättigt sind. Der Ausdruck umfasst auch Amine mit 2 oder mehreren Aminogruppen.

Der Ausdruck "Alkalimetall" umfasst Lethium, Natrium und Kalium und der Ausdruck "Erdalkalimetall" umfasst Magnesium, Calcium, Strontium und Barium.

Die im rahmen der vorliegenden Erfindung offenbarten Formeln von Polyenen umfassen jeweils alle cis/trans- bzw. E/Z-Isomeren oder Gemische hiervon, sofern nicht ausdrücklich etwas anderes erwähnt wird. Bevorzugt sind im allgemeinen die all-E-Isomere. Im Falle von Doppelbindungen, welche einen Sulfonylrest aufweisen entspricht die E-Form der cis-Konfiguration (bezüglich der Kohlenstoffreste), in den übrigen Fällen entspricht die E-Form der trans-Konfiguration.

Die erfindungsgamässe Umsetzung mit Dithionit in Gegenwart von Ammoniak oder einem aliphatischen Amin ermöglicht überraschenderweise die Herstellung der Verbindungen der Formel III in hoher Ausbeute ausgehend von den Sulfonylpolyenen der Formel I. Die Reduktion verläuft zudem in hoher Stereoselektivität. Beispielsweise werden ausgehend von all-E-Sulfonen der Formel I überwiegend die all-trans-Verbindungen der Formel III erhalten. Die Sulfone der Formel I sind kristalline Verbindungen und daher gut zu reinigen, was die Herstellung isomerenreiner Verbindungen der Formel III zusätzlich erleichtert.

Die Reduktion der Verbindungen der Formel I kann mit üblichen Dithioniten erfolgen, beispielsweise mit Natriumdithionit, Zinkdithionit, Bariumdithionit, Tetraalkylammoniumdithionit (z.B. Methyl-tridecylammoniumdithionit) und dergleichen. Vorzugsweise wird Alkalimetalldithionit, insbesondere Natriumdithionit verwendet. Das Dithionit wird zweckmässigerweise in mindestens etwa äquimolaren Mengen, vorzugsweise in einer Menge von etwa 1-5 Moläquivalenten oder mehr bezogen auf das Sulfon der Formel I verwendet.

Die Reduktion der Verbindungen der Formel I erfolgt in Gegenwart von Ammoniak oder einem aliphatischen Amin. Die Amine besitzen vorzugsweise einen pKa-Wert (gemessen in wässriger Lösung bei 20 °C) von mindestens etwa 9 und besondere bevorzugt von mindestens etwa 9,25 (pKa-Wert von Ammoniak). Dies ist für praktisch alle üblichersweise als aliphatische Amine bezeichneten Verbindungen erfüllt. Beispiele bevorzugter aliphatischer Amine sind die Alkylamine, wie Methylamin, Aethylamin, n-Butylamin oder t-Butylamin, die Dialkylamine, wie Dimethylamin oder Diäthylamin, die Trialkylamine, wie Trimethylamin oder Triäthylamin, die cyclishen Amine wie Pyrrolidin, Piperidin oder Chinuclidin, die Alkylendiamine, wie Aethylendiamin, Putrescin oder Hexamethylendiamin, die Aralkylamine, wie Benzylamin oder Phenäthylamin, die Hydroxylamine, wie N-Aethylhydroxylamin, N,N-Dimethylhydroxylamin oder N-Benzyl-N-methyl-hydroxylamin, und dergleichen. "Aralkyl" bedeutet hier vorzugsweise Phenylalkyl. Besonders bevorzugte Stickstoffbasen sind Ammoniak, die Alkylamine, die Dialkylamine, die Trialkylamine, die cyclischen aliphatischen Amine und die Alkylendiamine. Die Menge an Ammoniak oder Amin ist nicht kritisch; vorzugsweise wird jedoch in der Regel ein deutlicher Ueberschuss, beispielsweise etwa 5-30 Moläquivalente bezogen auf das Sulfon der Formel I, verwendet.

Die Reduktion wird zweckmässig in einem wässrig-organischen Lösungsmittelgemsich durchgeführt. Als organische Komponente des Lösungsmittelgemisches eignen sich inerte organische Lösungsmittel, beispielsweise Aether, wie Diäthyläther, Diisopropyläther, Tetrahydrofuran, Dioxan oder 1,2-Dimethoxyäthan, gesättigte oder aromatische Kohlenwasserstoffe, wie Hexan, Cyclohexan, Benzol oder Toluol, Amide wie Dimethylformamid und dergleichen. Bevorzugte organische Lösungsmittel sind die Aether, insbesondere Tetrahydrofuran und 1,2-Dimethoxyäthan. Temperatur und Druck sind nicht kritisch. Beispielsweise kann die erfindungsgemässe Reduktion bei Atmosphärendruck und einer Temperatur von etwa 0 °C bis Rückflusstemperatur, vorzugsweise bei etwa raumtemperatur bis 50 °C durchgeführt werden.

Im erhaltenen Produkt gegebenenfalls vorhandene Schutzgruppen können gewänschtenfalls nach bekannten Methoden abgespalten werden, beispielsweise durch Hydrolyse mit Säure oder Base.

Bei der erfindungsgemässen Reduktion wird eine cis-Doppelbindung an der Sulfongruppe (E-Form) fas ausschliesslich in eine trans-Doppelbindung übergeführt und umgekehrt, während die Konfiguration der übrigen Doppelbindungen erhalten bleibt. Gewünschtenfalls kann das Produkt nach bekannten Methoden isomerisiert werden, beispielsweise durch Erhitzen in Wasser oder einem organischen Lösungsmittel zwecks Erhöhung des Anteils an all-trans-Isomer. Die Reinigung des Produktes der Formel I kann in bekannter Weise erfolgen, beispielsweise auf chromatographischem Wege und/oder durch Umkristallisation.

Die Verbindungen der Formel I sind neu und ebenfalls Gegenstand der vorliegenden Erfindung.

Eine bevorzugte Gruppe von Verbindungen der Formel I sind diejenigen, worin entweder $X^2$ einen Sulfonylrest, $X^6$ Wasserstoff oder einen Sulfonylrest und $X^1$, $X^3$, $X^4$, $X^6$, $X^7$, $X^6$ und $X^6$ Wasserstoff bedeuten oder $X^4$ einen Sulfonylrest, $X^6$ Wasserstoff oder einen Sulfonylrest und $X^1$, $X^2$, $X^3$, $X^6$, $X^6$, $X^7$ und $X^6$ Wasserstoff bedeuten. Wenn $X^6$ oder $X^6$ einen Sulfonylrest bezeichnet, besitzt n die Bedeutung von m und $R^2$ die Bedeutung von $R^1$. Wenn $X^6$ und $X^6$ Wasserstoff bezeichnen, steht $R^2$ vorzugsweise für eine acetalisierte Formylgruppe oder für eine gegebenenfalls veresterte Carboxygruppe.

Eine weitere Gruppe bevorzugter Verbindungen der Formel I sind diejenigen, worin m und n für die Zahl 1 stehen, $R^1$ und $R^2$ 4-Methyl-3-pentenyl bezeichnen, entweder die beiden Gruppen $X^3$ und $X^7$ oder die beiden gruppen $X^6$ und $X^6$ Sulfonylreste darstellen und die übrigen der Gruppen $X^1$-$X^6$ jeweils Wasserstoff bedeuten.

Eine weitere Gruppe bevorzugter Verbindungen der Formel I sind diejenigen, worin $X^1$ einen Sulfonylrest und $X^2$-$X^9$ Wasserstoff bedeuten.

Die obige Formel I umfasst alle cis/trans-Isomeren sowie Gemische hiervon. Je nach Wahl des Isomers oder Isomerengemisches können unterschiedliche Isomere oder Isomerengemische der Verbindungen der Formel III erhalten werden. Besonders bevorzugt sind im allgemeinen die all-E-Sulfone der Formel I, welche direkt zu den wichtigen all-trans-Isomeren der Formel III führen.

Die all-E-Formen von bevorzugten Gruppen von Verbindungen der Formel I sind in den folgenden allgemeinen Formeln Ia-Id dargestellt:

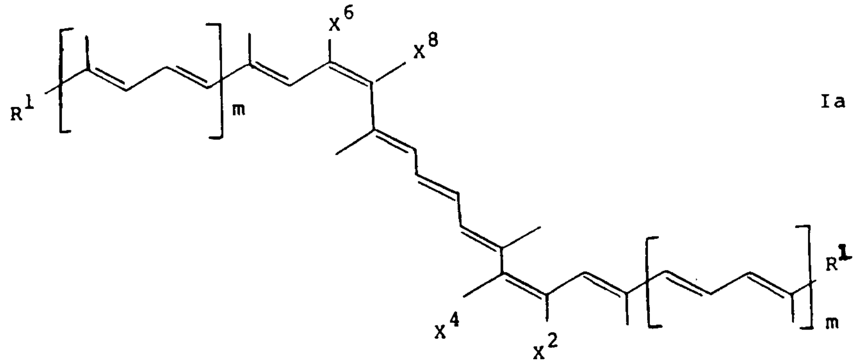

Ia

worin $X^2$ und $X^9$ Sulfonylreste und $X^4$ und $X^9$ Wasserstoff bezeichnen oder $X^2$ und $X^9$ Wasserstoff und $X^4$ und $X^9$ Sulfonylreste bezeichnen, und m und $R^1$ die obigen Bedeutungen haben;

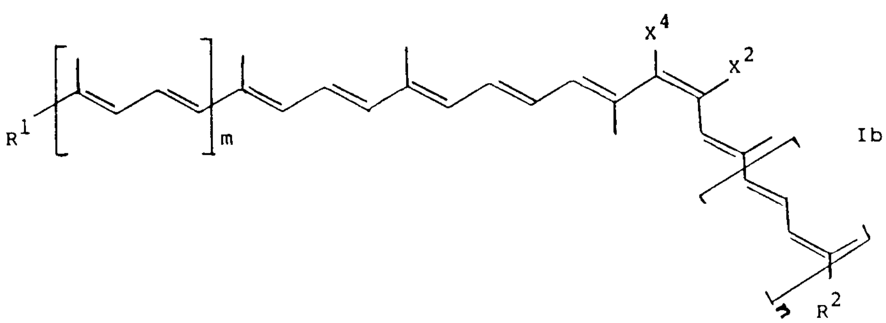

Ib

worin $X^2$ einen Sulfonylrest und $X^4$ Wasserstoff bezeichnen, oder $X^2$ Wasserstoff und $X^4$ einen Sulfonylrest bezeichnen, und m, n, $R^1$ und $R^2$ die obigen Bedeutungen haben;

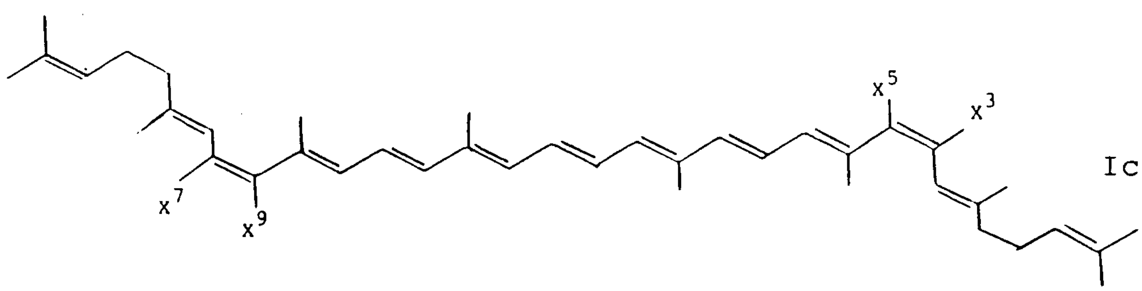

Ic

worin $X^3$ und $X^7$ Sulfonylreste und $X^9$ und $X^9$ Wasserstoff bezeichnen oder $X^3$ und $X^7$ Wasserstoff und $X^9$ und $X^9$ Sulfonylreste bezeichnen;

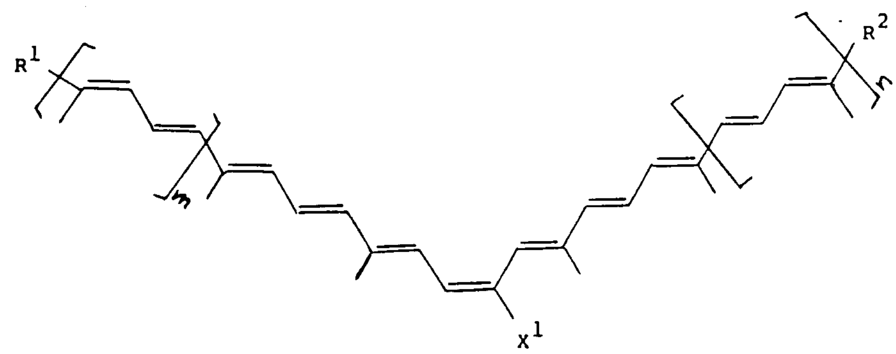

Id

worin $X^1$ einen Sulfonylrest bezeichnet und m, n, $R^1$ und $R^2$ die obigen Bedeutungen haben.

Im allgemeinen sind diejenigen Verbindungen der Formeln I, Ib und Id bevorzugt, worin $R^1$ und $R^2$ die gleiche Bedeutung haben oder $R^2$ eine acetalisierte Formylgruppe bedeutet. Ferner sind im allgemeinen diejenigen Verbindungen der Formel I, Ia, Ib und Ic bevorzugt, worin $X^1$, $X^2$ oder $X^3$ einen Sulfonylrest bedeutet.

In obiger Former II steht vorzugsweise $R^3$ für Wasserstoff oder einen gegbenenfalls geschützte Hydroxypgruppe, insbesondere für Wasserstoff, Hydroxy, Acetoxy, Methoxy oder 1-Methoxy-1-methyläthoxy. $R^4$ steht vorzugsweise für Wasserstoff oder eine geschützte Oxogruppe, insbesondere für Wasserstoff, für Aethylendioxy oder für 2 Methoxygruppen.

Die Verbindungen der Formel I können erfindungsgemäss dadurch hergestellt werden, dass man

a) eine Verbindung der allgemeinen Formel

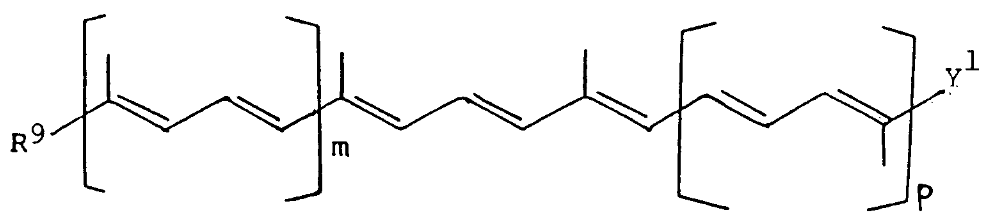

V

mit einer Verbindung der allgemeinen Formel

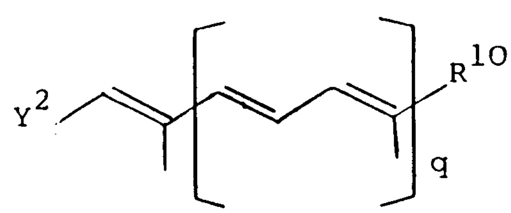

VI

oder

b) eine Verbindung der allgemeinen Formel

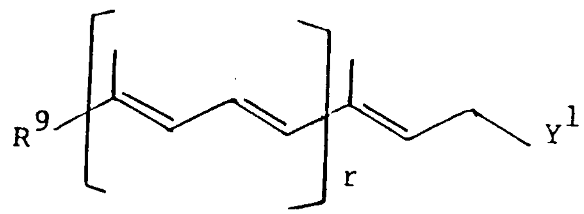

VII

mit einer Verbindung der allgemeinen Formel

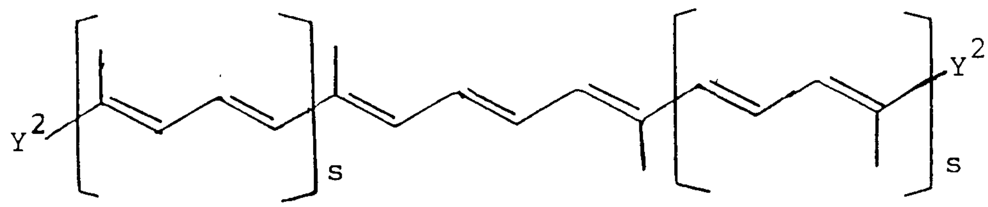

VIII

worin eine der Gruppen $Y^1$ und $Y^2$ Formyl und die andere eine Gruppe $-Ch_2X$ darstellt; X einen Sulfonylrest darstellt; p und q für die Zahlen 0, 1 oder 2 stehen und p + q = n + 1 beträgt; r und s für die Zahlen 0 oder 1 stehen und r + s = m beträgt; m die Zahl 1 und $R^9$ 4-Methyl-3-pentenyl bezeichnet, oder m die Zahl 0 und $R^9$ eine acetalisierte Formylgruppe, eine gegebenenfalls veresterte Carboxygruppe oder eine Gruppe der allgemeinen Formel

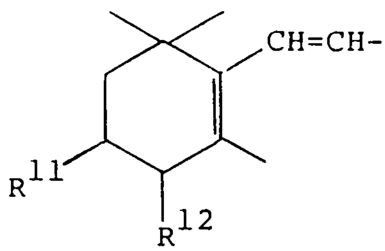

IX

bezeichnet; n die Zahl 1 und $R^{10}$ 4-Methyl-3-pentenyl bezeichnet, oder n die Zahl 0 und $R^{10}$ eine acetalisierte Formylgruppe, eine gegebenenfalls veresterte Carboxygruppe oder eine Gruppe der Formel IX bezeichnet; und $R^{11}$ und $R^{12}$ unabhängig voneinander Wasserstoff, eine gegebenenfalls geschützte Hydroxygruppe oder eine geschützte Oxogruppe darstellen,
in einem inerten organischen Lösungsmittel in Gegenwart einer Base umsetzt und die nach Zugabe eines Acylierungsmittels erhaltene Verbindung der allgemeinen Formel

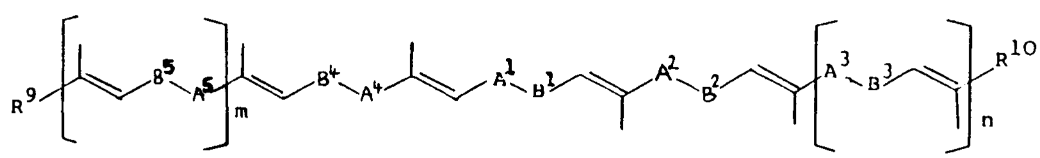

X

worin eine der Gruppen $-A^1-B^1-$, $-A^2-B^2-$ und $-A^3-B^3-$ die Gruppe -CHX-CH(OR)- oder -CH(OR)-CHX- darstellt und die beiden andern -CH=CH- darstellen; $-A^4-B^4-$ und $-A^5-B^5-$ für -CH=CH- stehen oder, wenn $R^{10}$ die Bedeutung von $R^9$ und n die Bedeutung von m hat, auch $-A^4-B^4-$ die Bedeutung von $-A^2-B^2-$ und $-A^5-B^5-$ die Bedeutung von $-A^3-B^3-$ hat; X einen Sulfonylrest darstellt; -OR Acyloxy bezeichnet; $R^9$, $R^{10}$, m und n die obigen Bedeutung haben; und $R^{11}$ und $R^{12}$ unabhängig voneinander Wasserstoff, eine geschützte Hydroxygruppe oder eine geschützte Oxogruppe darstellen,
mit wässriger Base in eine Verbindung der Formel I überführt.

Die Umsetzung einer Verbindung der Formel V mit einer Verbindung der Formel VI und die Umsetzung einer Verbindung der Formel VII mit einer Verbindung der Formel VIII können in an sich bekannter Weise in einem inerten organischen Lösungsmittel erfolgen. Als Deprotonierungsmittel können übliche Basen verwendet werden, beispielsweise Alkylderivate von Alkalimetallen, Grignard-Reagenzien, Alkalimetallamide, Alkalimetallhydride und dergleichen, wie Methyllithium, Butyllithium, Aethylmagnesiumbromid, Diäthylmagnesium, Lithiumdiisopropylamid und Natriumhydrid. Beispiele geeigneter Lösungsmittel sind die Aether, wie Diäthyläther, Diisopropyläther, t-Butylmethyläther, Tetrahydrofuran, Dioxan und 1,2-Dimethoxyäthan, die gesättigten und aromatischen Kohlenwasserstoffe, wie Hexan, Cyclohexan, Benzol und Toluol, und dergleichen. Temperatur und Druck sind nicht kritisch. Im allgemeinen wird die Umsetzung jedoch bei Atmosphärendruck und einer Temperatur von etwa -80 °C bis Raumtemperatur, vorzugsweise bei einer Temperatur von etwa -70 °C bis etwa -15 °C durchgeführt.

Aus dem Reaktionsgemisch können in der Regel keine β-Hydroxysulfone isoliert werden. Es ist daher wichtig, dass dem Reaktionsgemisch ohne vorherige Aufarbeitung ein Acylierungsmittel zugesetzt wird. Es wurde gefunden, dass die β-Acyloxysulfone der Formel X in guter Ausbeute hergestellt und, gewünschtenfalls, aus dem Reaktionsgemisch isoliert werden können. Zudem können die Verbindungen der Formel X leicht in Verbindungen der Formel I übergeführt werden.

Die Umsetzung zum Acyloxyderivat der Formel X kann mit üblichen Acylierungsmitteln, beispielsweise mit Acylanhydrid, Acylchlorid oder Acylbromid, erfolgen. Der Ausdruck "Acyl" besitzt hier vorzugsweise die gleiche Bedeutung wie in den eingangs genannten Acyloxygruppen. Besonders bevorzugt ist die Herstellung der Acetoxyderivate der Formel X mit Acetanhydrid. Das Acylierungsmittel wird vorzugsweise im Ueberschuss eingesetzt. Temperatur und Druck sind nicht kritisch. Im allgemeinen wird jedoch die Acylierung bei Atmosphärendruck und einer Temperatur von etwa -70 °C bis Raumtemperatur, vorzugsweise bei etwa -20 °C bis etwa +10 °C durchgeführt. In $R^9$ und/oder $R^{10}$ gegebenenfalls vorhandene freie Hydroxygruppen werden bei dieser Umsetzung ebenfalls acyliert.

Die Umsetzung einer Verbindung der Formel X zu einer Verbindung der Formel I kann zweckmässigerweise mit wässriger Base erfolgen. Vorzugsweise wird die Umsetzung mit Alkalimetallhydroxid oder Erdalkalimetallhydroxid in Wasser durchgeführt. Besonders bevorzugt ist Natronlauge oder Kalilauge. Gewünschtenfalls kann dem Reaktionsgemisch ein inertes organisches Lösungsmittel zugesetzt werden. Beispiele geeigneter organischer Lösungsmittel sind die oben, im Zusammenhang mit der Herstellung der Verbindungen der Formel X genannten Lösungsmittel, insbesondere die Aether, Temperatur und Druck sind nicht kritisch. Im allgemeinen wird jedoch bei Atmosphärendruck und einer Temperatur von etwa 0 °C bis Rückflusstemperatur, vorzugswise bei etwa 0 °C bis Raumtemperatur gearbeitet. In $R^9$ und/oder $R^{10}$ gegenbenenfalls vorhanene Estergruppen können bei der Umsetzung mit wässriger Base erhalten bleiben oder verseift werden, je nach verwendeter Base, Konzentration der Base, Reaktionstemperatur, Reaktionszeit und dergleichen.

Die Herstellung der Verbindung der Formel I kann vorzugsweise auch als Eintopf-Verfahren ohen Isolierung der Verbindungen der Formel X durchgeführt werden. Gemäss dieser Variante kann nach beendeter Acylierung die wässrige Base direkt zum Reaktionsgemisch zugesetzt werden.

Die Konfiguration der Doppelbindungen in den Verbindungen der Formeln V-VIII bleibt bei der Umsetzung zu Verbindungen der Formeln X und I vorwiegend erhalten. Die mit einem Sulfonylrest substituierten Doppelbindungen in Formel I werden im allgemeinen überwiegend in der E-Form gebildet. Ausgehend von den trans-Isomeren bzw. den all-trans-Isomeren der Verbindungen der Formeln V und VI oder der Verbindungen der Formeln VII und VIII können somit direkt vorwiegend die bevorzugten all-trans-Isomere der Verbindungen der Formel X une die bevorzugten all-E-Isomere der Verbindungen der Formel I erhalten werden.

Die Verbindungen der Formel X sind neu und ebenfalls Gegenstand der vorliegenden Erfindung. Formel X umfasst die durch Umsetzung einer Verbindung der Formel V mit einer Verbindung der Formel VI erhältlichen Verbindungen der allgemeinen Formel

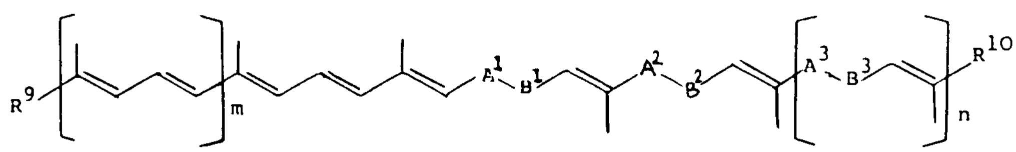

Xa

worin $R^9$, $R^{10}$, m, n, $-A^1-B^1-$, $-A^2-B^2-$ und
$-A^3-B^3-$ die in Formel X gegebenen Bedeutungen haben,
und die durch Umsetzung einer Verbindung der Formel VII mit einer Verbindung der Formel VIII erhältlichen Verbindungen der allgemeinen Formel

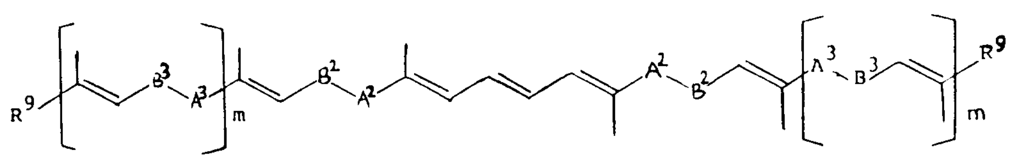

Xb

worin $-A^2-B^2-$die Gruppe -CHX-CH(OR)- oder -CH(OR)-CHX- und $-A^3-B^3-$ die Gruppe -CH = CH-darstellen, oder $-A^2-B^2-$ die Gruppe -CH = CH- und $-A^3-B^3-$ die Gruppe -CHX-CH(OR)- oder -CH(OR)-CHX-darstellen; und $R^9$, m, X und R die in Formel X gegebenen Bedeutungen haben.

Bevorzugte Verbindungen der Formeln X, Xa und Xb sind diejenigen, welche zu bevorzugten Verbindungen der Formel I führen. Bevorzugt sind beispielsweise die all-trans-Isomere sowie diejenigen Verbindungen, worin eine der Gruppen $-A^1-B^1-$, $-A^2-B^2-$ und $-A^3-B^3-$ die Gruppe -CHX-CH(OR)- darstellt. Bevorzugte Sulfonylreste X, Acyloxygruppen -OR und Schutzgruppen sind die eingangs genannten Gruppen.

Die als Ausgangsmaterialien verwendeten Verbindungen der Formeln V-VIII sind bekannte Verbindungen oder können leicht nach an sich bekannten Methoden hergestellt werden.

Die Erfindung wird durch die folgenden Beispiele weiter veranschaulicht.

Beispiel 1

a) In einem Rundkolben mit mechanischem Rührer, Tropftrichter, Thermometer und Argonbegasung wurden 90,9 g (240 mMol) 5-(2,6,6-Trimethyl-1-cyclohexenyl)-3-methyl-2E,4E-pentadienyl -(p-chlorphenyl)sulfon in 500 ml tetrahydrofuran gelöst und die Lösung auf -70 °C gekühlt (Aethanol/Trockeneis-Bad). Anschliessend wurden innert 15 Minuten 150 ml einer 1,6M Lösung von Butyllithium (240 mMol) in Hexan und dann innert 20 Minuten eine Lösung von 16,4 g (100 mMol) (all-E)-2,7-Dimethyl-2,4,6-octatriendial in 800 ml Tetrahydrofuran zur Reaktionslösung zugetropft. Das Gemisch wurde noch 3 Stunden bei -60 °C gerührt und dann innert 10 Minuten tropfenweise mit 136 ml (1,4 Mol) Acetanhydrid versetzt, wobei die Temperatur deutlich anstieg. Das Reaktionsgemisch enthaltend (all-E)-12,12′-Diacetoxy-11,11′-bis[(p-chlorphenyl)sulfonyl] -11,12,11′,12′-tetrahydro-β,β-carotin wurde noch 2 Stunden bei 0-5 ° (Eisbad) gerührt, danach innert 20 Minuten mit 600 ml 28-prozentiger wässriger Natronlauge (5,5 Mol) versetzt und über Nacht ohen Kühlung weitergerührt. Anschliessend wurde das Reaktionsgemisch auf Eiswasser gegossen und mit Methylenchlorid extrahiert. Die organische Phase wurde mit 5-prozentiger Kaliumdihydrogenphosphat-Lösung und mit Wasser neutral gewaschen und am Rotationsverdampfer auf eine Menge von ca. 1 kg eingeengt. Aus der erhaltenen organen Suspension wurde Lösungsmittel und Wasser abdestilliert und gleichzeitig Diisopropyläther zugetropft, bis ein Siedepunkt von 66 °C erreicht war (Lösungsmittelaustausch). Die Suspension (ca. 500 ml) wurde über Nacht im Kühlschrank aufbewahrt und dann genutscht. Die Kristalle wurden bei 45 °C im Wasserstrahlvakuum getrocknet. Hierbei wurden 74,5 g (84%) (all-E)-β,β-carotin-11,11′-ylen-bis[(p-chlorphenyl)sulfon] als orange Kristalle mit Smp. 172-173 °C und einem Gehalt an all-E-Isomer von 94% erhalten. Das durch Eindampfen der Mutterlauge erhaltene Oel (36 g), welches gemäss chromatographischer Analyse 35% Produkt enthielt, wurde nicht aufgearbeitet.

b) In einem Sulfierkolben mit mechanischem Rührer, Thermometer und Argonbegasung wurden 3,54 g (4 mMol) (all-E)-β,β-Carotin-11,11′-ylen-bis[p-chlorphenyl)sulfon] in 354 ml 1,2-Dimethoxyäthan bei Raumtemperatur gelöst. Diese Lösung wurde zuerst mit 7,5 ml 25-prozentigem wässrigem Ammoniak (100 mMol) und dann unter Kühlung (Eisbad) mit einer Lösung von 7,0 g 85-prozentigem Natriumdithionit (34 mMol) in 100 ml Wasser versetzt. Dabei stieg die Innentemperatur auf 30 °C. Das gemisch wurde noch 5 Stunden bei 25-30 °C gerührt und dann in einen Scheidetrichter auf 200 ml Wasser und 250 ml Methylenchlorid gegossen. Die organische Phae wurde mit 200 ml 5-prozentiger wässriger Kaliumdihydrogenphosphat-Lösung und zweimal mit je 100 ml Wasser gewaschen, über Natriumsulfat getrocknet, filtriert und eingedampft. Hierbei wurden 2,17 g rohes β-Carotin als violett glänzender, fester Rückstand erhalten. Das Rohprodukt wurde in Methylenchlorid gelöst und dann unter gleichzeitigem Abdestillieren des Methylenchlorids tropfenweise mit Methanol versetzt. Die erhaltene Suspension in Methanol wurde noch 1 Stunde am Rückfluss gekocht und dann auf 0 °C abgekühlt. Filtration und trocknung des Rückstandes im Hochvakuum ergab 1,92 g (90%) schwarzviolettes, kristallines β-Carotin mit Smp. 170-174 °C, welches 90% all-E-Isomer und 3% 9Z-Isomer enthielt. Das durch Einengen der Mutterlauge erhaltene Oel (0,26 g) wurde nicht aufgearbeitet.

In einem analogen Ansatz wurde das Reaktionsgemisch enthaltend (all-E)-12,12′-Diacetoxy-11,11′-bis[p-chlorphenyl)sulfonyl] -11,12,11′,12′-tetrahydro-β,β-carotin aufgearbeitet durch Extraktion mit Phosphatpuffer-Lösung (pH 7), Wasser und t-Butylmethyläther. Die organischen Phasen wurden über Natriumsulfat getrocknet, filtriert und eingeengt. Umkristallisation des erhaltenen, weissen Kristallpulvers aus Methylenchlorid und Diäthyläther ergab (all-E)-12,12′-Diacetoxy-11,11′-bis[(p-chlorphenyl)sulfonyl] -11,12,11′,12′-tetrahydro-β-,β-carotin mit Smp. 178-179 °C (Zersetzung ab 160 °C).

Beispiel 2

a) In einem Runkolben mit mechanischem Rührer, Tropftrichter, Thermometer und Argonbegasung wurden 22,4 g (65 mMol) 5-(2,6,6-Trimethyl-1-cyclohexenyl)3-methyl-2E,4E-pentadienyl -phenyl-sulfon in 125 ml Tetrahydrofuran gelöst und die Lösung auf -70 °C gekühlt (Aethanol/Trockeneis-Bad). Anschliessend wurden innert 15 Minuten 40,6 ml einer 1, 6M Lösung von Butyllithium (65 mMol) in Hexan und dann innert 20 Minuten eine Lösung von 4,1 g (25 mMol) (all-E)-2,7-Dimethyl-2,4,6-octatriendial in 200 ml Tetrahydrofuran zur Reaktionslösung zugetropft. Das Gemisch wurde noch 3 Stunden bei -60 °C gerührt und dann innert 10 Minuten tropfenweise mit 37,4 ml (0,4 Mol) Acetanhydrid versetzt, wobei die Temperatur deutlich anstieg. Das Reaktionsgemisch enthaltend (all-E)-12,12′-Diacetoxy-11,11′-bis-(phenylsulfonyl) -11,12,11′,12′-tetrahydro-β,β-carotin wurde noch 2 Stunden bei 0-5° (Eisbad) gerührt, danach bei -5 °C rasch mit 165 ml 28-prozentiger wässriger Natronlauge (1,52 Mol) versetzt und über Nacht ohne Kühlung weitergerührt. Anschliessend wurde das Reaktionsgemisch mit 5-prozentiger wässriger Kaliumdihydrogenphosphat-Lösung und einem Gemisch von Diäthyläther und Methylenchlorid (Vol. 5:3) extrahiert. Die organische Phase wurde über Natriumsulfat getrocknet, filtriert und eingeengt. Das erhaltene Rohprodukt wurde in 200 ml Methylenchlorid gelöst. Die Lösung wurde am Rotationsverdampfer auf eine Menge von 100 g eingeengt, dann mit 100 ml Diisopropyläther versetzt und erneut auf 100 g eingeengt, wobei Produkt auszufallen begann. Diese Suspension wurde nochmals mit 100 ml Diisopropyläther versetzt, dann auf 100 g eingeengt und zur vollständigen Kristallisation 3 Stunden bei 4 °C aufbewahrt. Filtration und Trocknung der Kristalle am Hochvakuum ergab 14,3 g (70%) (all-E)-β,β-Carotin-11,11′-ylen-bis(phenylsulfon) als oranges Pulver mit Smp. 190-191 °C, welches 92% all-E-Isomer und 8% andere Isomere enthielt. Das durch Einengen der Mutterlauge erhaltene Oel (13,2 g), welches weiteres Produkt als Isomerengemisch enthielt wurde nicht aufgearbeitet.

b) Das erhaltene (all-E)-β,β-Carotin-11,11′-ylen-bis(phenylsulfon) wurde in analoger weise zu Beispiel 1b zu β-Carotin umgesetzt.

Beispiel 3

a) In einem Mehrhalskolben mit Magnetrührer und Thermometer wurden unter Inertgas 890 mg (2 mMol) (all-E)-9-(2,6,6-Trimethyl-1-cyclohexenyl)-3,7-dimethyl -2,4,6,8-nonatetraenyl-(p-chlorphenyl)sulfon [Retinyl-(p-chlorphenyl)sulfon] in 3 ml Tetrahydrofuran gelöst. Die Lösung wurde auf -70 °C gekühlt und anschliessend tropfenweise zuerst innert 15 Minuten mit 1,25 ml einer 1,6M Lösung von Butyllithium (2 mMol) in Hexan und dann innert 15 Minuten mit einer Lösung von 284,4 mg (1 mMol) Vitamin-A-aldehyd (Retinal) in 4 ml Tetrahydrofuran versetzt. Das Reaktionsgemisch wurde noch 4 Stunden bei -70 °C gerührt und dann mit 0,756 ml (8 mMol) Acetanhydrid versetzt. Das Gemisch wurde noch 2 stunden bei 0 °C und dann über Nacht bei Raumtemperatur gerührt. Danach wurde das Reaktionsgemisch enthaltend (all-E)-15′-Acetoxy-15-(p-chlorphenyl)sulfonyl -15,15′-dihydro-β,β-carotin unter guter Kühlung bei 0-5 °C rasch mit 3,5 ml 28-prozentiger wässriger Natronlauge (32 mMol) versetzt und noch 6,5 Stunden bei Raumtemperatur gerührt. Danach wurde das Reaktionsgemisch mit Wasser und mit 5-prozentiger Kaliumdihydrogenphosphat-Lösung extrahiert. Die Waschlösungen wurden mit Methylenchlorid nachextrahiert. Die vereinigten organischen Phasen wurden über Natriumsulfat getrocknet, filtriert und am rotationsverdampfer eingeengt. Hierbei wurden 1,1 g (all-E)-β,β-Carotin-15-yl-(p-chlorphenyl)sulfon erhalten, welches ohne weitere Reinigung in der nächsten Stufe verwendet wurde.

b) Das erhaltene (all-E)-β,β-Carotin-15-yl-(p-chlorphenyl)sulfon (1,1 g) wurde in 12,5 ml tetrahydrofuran gelöst. Diese Lösung wurde mit 1,3 ml (12,5 mMol) Diäthylamin und mit einer Lösung von 0,875 g 85-prozentigem Natriumdithionit (4,27 mMol) in 12,5 ml Wasser verstezt und noch 7,5 Stunden bei Raumtemperatur gerührt. Danach wurde das Reaktionsgemisch mit 5-prozentiger wässriger Kaliumdihydrogenphosphat-Lösung und mit 2-prozentiger wässriger Kochsalzlösung extrahiert. Die Waschlösungen wurden mit Methylenchlorid nachextrahiert. Die organischen Phasen wurden vereinigt und eingeengt. Das erhaltene rote Oel wurde in 100 ml Wasser aufgenommen une 21 Stunden zum Rückfluss erhitzt. Danach wurde das Gemisch genutscht, wobei 850 mg Rohprodukt als dunkelrotes Pulver zurückblieben. Dieses Rohprodukt wurde in 160 ml Cyclohexan digeriert und der unlösliche Rückstand abgenutscht. Das Filtrate wurde an Kieselgel mit Cyclohexan/Toluol (Vol. 9:1) chromatographisch getrennt. Einengen der produkthaltigen Fraktionen und Trocknung am Hochvakuum ergab 280 mg β-Carotin mit Smp. 170-171 °C und einem Gehalt an all-E-Isomer von 90%.

Beispiel 4

a) In einem Mehrhalskolben mit Magnetrührer und Thermometer wurden unter Inertgas 2,5 g (6 mMol) (all-E)-9-(2,6,6-Trimethyl-1-cyclohexenyl)-3,7-dimethyl -2,4,6,8-nonatetraenyl-phenyl-sulfon [Retinyl-phenylsulfon] in 9 ml Tetrahydrofuran gelöst. Die Lösung wurde auf -60 °C gekühlt und anschliessend tropfenweise zuerst innert 10 Minuten mit 3,72 ml einer 1,6M Lösung von Butyllithium (6 mMol) in Hexan und dann innert 10 Minuten mit einer Lösung von 853 mg (3 mMol) Vitamin-A-Aldehyd (Retinal) in 12 ml Tetrahydrofuran versetzt. Das Reaktionsgemisch wurde noch 70 Minuten bei -60 °C gerührt und dann mit 2,27 ml (24 mMol) Acetanhydrid versetzt. Das Gemisch wurde noch 1,5 Stunden bei 0 °C gerührt. Danach wurde das Reaktionsgemisch enthaltend (all-E)-15′-Acetoxy-15-phenylsulfonyl-15,15′-dihydro -β,β-carotin unter guter Kühlung bei 0-5 °C rasch mit 10,5 ml 28-prozentiger wässriger Natronlauge (96 mMol) versetzt und noch 16 Stunden bei Raumtemperatur gerührt. Danach wurde das Reaktionsgemisch mit Wasser und mit 5-prozentiger Kaliumdihydrogenphosphat-Lösung extrahiert. Die Waschlösungen wurden mit Diäthyläther nachextrahiert. Die vereinigten organischen Phasen wurden über Natriumsulfat getrocknet, filtriert und am Rotationsverdampfer eingeengt. Hierbei wurden 3,07 g Rohprodukt als rotes Oel erhalten. Chromatographische Trennung an Kieselgel mit Hexan/Aceton (Vol. 1:1) ergab 1,41 g (all-E)-β,β-Carotin-15-yl-phenylsulfon.

b) 338,5 g (0,5 mMol) (all-E)-β,β-carotin-15-yl-phenylsulfon wurden in 6,25 ml Tetrahydrofuran gelöst. Diese Lösung wurde mit 0,65 ml (6,25 mMol) Diäthylamin und mit einer Lösung von 0,44 g 85-prozentigem Natriumdithionit (2,14 mMol) in 6,25 ml Wasser versetzt und noch 3 Tage bei Raumtemperatur gerührt. Danach wurde das Reaktionsgemisch mit Tetrahydrofuran/Diäthyläther extrahiert. Die organischen Phasen wurden mit 5-prozentiger Kaliumdihydrogenphosphat-Lösung und mit 1-prozentiger Kochsalzlösung gewaschen, über Natriumsulfat getrocknet, filtriert und eingeengt. Das erhaltene Rohprodukt (255 mg) wurde mit 2,5 ml Methylenchlorid und 20 ml Hexan versetzt. Diese Suspension wurde genutscht und der Rückstand mit Hexan gewaschen und am Hochvakuum getrocknet. Hierbei wurden 176 mg β-Carotin als dunkelrotes kristallines Pulver mit Smp. 180-181 °C und einen Gehalt an all-E-Isomer von 94% erhalten.

Beispiel 5

a) Eine Lösung von 2,5 g (8 mMol) 3,7-Dimethyl-2E,6E-octadienyl-(p-chlorphenyl)sulfon in 16 ml Tetrahydrofuran wurde bei -70 °C tropfenweise zuerst innert 10 Minuten mit 5 ml einer 1,6M Lösung von Butyllithium (8 mMol) in Hexan und dann innert 30 Minuten mit einer Lösung von 593 mg (2 mMol) Crocetindialdehyd [(all-E)-2,6,11,15-Tetramethyl-2,4,6,8,10,12,14 -hexadecaheptaendial] in 100 ml Tetrahydrofuran versetzt. Die Suspension wurde noch 21 Stunden bei -70 °C gerührt, dann mit 3 ml (31,7 mMol) Acetanhydrid versetzt und noch 8 Stunden bei 0 °C gerührt. Danach wurde das rote Reaktionsgemisch enthaltend (all-E)-8,8′-Diacetoxy-7,7′-bis[p-chlorphenyl)sulfonyl] -7,8,7′,8′-tetrahydrolycopin unter guter Kühlung bei 0-10 °C mit 13 ml 28-prozentiger wässriger Natronlauge (120 mMol) versetzt und noch 40 Stunden bei Raumtemperatur gerührt. Zur Aufarbeitung wurde das Reaktionsgemisch mit Tetrahydrofuran und Wasser verdünnt. Die organische Phase wurde abgetrennt und mit 50 ml 5-prozentiger Kaliumdihydrogenphosphat-Lösung und mit 50 ml 2-prozentiger Kochsalzlösung gewaschen. Die Waschlösungen wurden zweimal mit je 30 ml Hexan/Methylenchlorid (Vol. 2:1) nachextrahiert. Die organischen Phasen wurden ohne Trocknung eingeengt. Das erhaltene, rohe (all-E)-Lycopin-7,7′-ylen-bis[(p-chlorphenyl)sulfon] wurde ohne weitere Reinigung in der nächsten Stufe verwendet.

Durch chromatographische Trennung eines auf analoge Weise erhaltenen Rohproduktes an Kieselgel mit Cyclohexan/Methylenchlorid/Diäthyläther (Vol. 4:1:1) und Umkristallisation wurde reines (all-E)-Lycopin-7,7′-ylen-bis[(p-chlorphenyl)sulfon] erhalten; Smp. 179-180 °C.

b) Das erhaltene, rohe (all-E)-Lycopin-7,7′-ylen-bis[(p-chlorphenyl)sulfon] wurde in 177 ml Tetrahydrofuran aufgenommen. Die Lösung wurde bei -5 °C bis +5 °C mit 5,2 ml (50 mMol) Diäthylamin und mit einer Lösung von 3,5 g 85-prozentigem Natriumdithionit (17 mMol) in 50 ml Wasser versetzt und noch 3 Tage bei Raumtempratur gerührt. Danach wurde das Reaktionsgemisch mit 5-prozentiger wässriger Kaliumdihydrogenphosphat-Lösung und mit 2-prozentiger wässriger Kochsalzlösung extrahiert. Die Waschlösungen wurden mit Aethylacetat nachextrahiert. Die organischen Phasen wurden vereinigt und eingeengt. Das erhaltene Rohprodukt wurde in 200 ml Wasser aufgenommen und 10 Stunden zum Rückfluss erhitzt. Anschliessend wurde das gemisch abgekühlt und genutscht. Der Rückstand wurde mit Wasser gewaschen und aus einem Gemisch von Methylenchlorid und Methanol umkristallisiert. Hierbei wurden 884 mg Lycopin (psi,psi-Carotin) als violette Plättchen erhalten, welches 92% all-trans-Isomer und 4% 5-cis-Isomer enthielt. Durch Aufschlämmen in Methylenchlorid, Filtration und Trocknen wurde

Lycopin mit Smp. 165-166 ° C und einem Gehalt an all-trans-Isomer von 98% erhalten.

Beispiel 6

a) Eine Lösung von 6,1 g (20 mMol) 1-[4(R)-(1-Methoxy-1-methyläthoxy)-2,6,6-trimethyl -1-cyclohexenyl]-3-methyl-1E, 4-pentadien-3-ol in 20 ml Hexan wurde bei -5 ° C bis 0 ° C unter Rühren tropfenweise mit einer Losung von 6,6 ml (40 mMol) Benzolsulfinsäure-Natriumsalz in 30 ml Essigsäure/Wasser (Vol. 80:20) versetzt. Das Gemisch wurde noch 23 Stunden bei -5 ° C bis 0 ° C und dann 3 Stunden bei 50 ° C gerührt. Anschliessend wurde das abgekühlte Reaktionsgemisch auf 50 ml Aethylacetat und 100 ml Wasser gegossen. Die wässrige Phase wurde abgetrennt und viermal mit je 25 ml Aethylacetat nachextrahiert. Die organischen Phasen wurden dreimal mit je 100 ml 2-prozentiger Kochsalzlösung, zweimal mit je 50 ml halbgesättigter Natriumhydrogencarbonat-Lösung und noch einmal mit 50 ml 2-prozentiger Kochsalzlösung gewaschen, dann über Natriumsulfat getrocknet, filtriert und eingeengt. Trocknung des erhaltenen Oeles am Hochvakuum bei 35 ° C ergab 6,67 g (92,5%) 5-[4(R)-Hydroxy-2,6,6-trimethyl-1-cyclohexenyl] -3-methyl-2E,4E-pentadienyl-phenylsulfon als gelbbraunes Harz.

b) In einem Sulfierkolben wurde unter Argonbegasung eine Lösung von 6,67 g (18,5 mMol) 5-[4(R)-Hydroxy-2,6,6-trimethyl-1-cyclohexenyl] -3-methyl-2E,4E-pentadienyl-phenylsulfon in 37 ml Tetrahydrofuran vorgelegt. die Lösung wurde auf -60 ° C gekühlt und unter Rühren tropfenweise zuerst innert 20 Minuten mit 23,3 ml einer 1,6M Lösung von Butyllithium (37,3 mMol) in Hexan und dann innert 15 Minuten mit einer Lösung von 759 mg (4,6 mMol) (all-E)-2,7-Dimethyl-2,4,6-octatriendial in 37 ml Tetrahydrofuran versetzt. Das Reaktionsgemisch wurde noch 4 Stunden bei -70 ° C gerührt, dann mit 16,6 ml (176 mMol) Acetanhydrid versetzt und über Nacht bei -5 ° C gerührt. Anschliessend wurde das gelbe Reaktionsgemisch enthaltend (all-E)-3(R),12,3′(R),12′-Tetraacetoxy-11,11′-bis(phenylsulfonyl)-11,12,11′,12′-tetrahydro-zeaxanthin unter guter Kühlung bei 0-10 ° C mit 98 ml (900 mMol) 28 prozentiger wässriger Natronlauge versetzt und bei Raumtemperatur weiter gerührt. Zur Aufarbeitung wurde das Reaktionsgemisch mit 250 ml Methylenchlorid und mit 250 ml Wasser extrahiert. Die organische Phase wurde abgetrennt und mit 125 ml 5-prozentiger Kaliumdihydrogenphosphat-Lösung und mit 125 ml Wasser gewaschen. Die wässrigen Phasen wurden mit Methylenchlorid nachextrahiert. Die organischen Phasen wurden vereinigt, über Natriumsulfat getrocknet, filtriert und eingeengt. Hierbei wurden 9,2 g Rohprodukt als rotes Oel erhalten, welches neben Edukt zur Hauptsache aus (all-E)-R,R′-Zeaxanthin-11,11′-ylen-bis-(phenylsulfon) und dessen Monoacetat und dessen Diacetat im Verhältnis von ca. 1:2:7 bestand.

c) Das erhaltene Rohprodukt (9,2 g) wurde unter Argonbegasung in 800 ml 1,2-Dimethoxyäthan und 24,5 ml (236 mMol) Diäthylamin gelöst. Diese Lösung wurde bei etwa 25 ° C mit einer Lösung von 16,1 g 85-prozentigem Natriumdithionit (78,6 mMol) in 230 ml Wasser versetzt. Das Reaktionsgemisch wurde noch 45 Minuten bei 25-30 ° C gerührt und dann auf 400 ml Eiswasser und 500 ml Methylenchlorid gegossen. Die organische Phase wurde abgetrennt und mit 400 ml 5-prozentiger Kaliumdihydrogenphosphat-Lösung und zweimal mit je 200 ml Wasser gewaschen. Die wässrigen Phasen wurden zweimal mit je 200 ml Methylenchlorid nachextrahiert. Die organischen Phasen wurden vereinigt, über Natriumsulfat getrocknet, filtriert und eingeengt. Hierbei wurden 7,6 g halb kristallines Rohprodukt erhalten, welches (all-E)-R,R′-Zeaxanthin und dessen Monoacetat und dessen Diacetat im Verhältnis von ca, 1:2:7 enthielt.

d) Eine Lösung des erhaltenen Rohproduktes (7,6 g) in 77 ml Methanol wurde mit 1,5 g (1,1 mMol) festem Kaliumcarbonat versetzt und über Nacht bei Raumtemperatur gerührt. Anschliessend wurde das Gemisch genutscht (Nachwaschen mit wenig Methanol) und das Filtrat (Mutterlauge I) aufbewahrt. Der Nutschkuchen bestehend aus Kaliumcarbonat und Zeaxanthin wurde in Wasser und Methylenchlorid gelöst. Die organische Phase wurde abgetrennt, über Natriumsulfat getrocknet, filtriert und eingeengt. Trocknung des erhaltenen Pulvers über Nacht am Hochvakuum bei Raumtemperatur ergab 500 mg (all-E)-R,R′-Zeaxanthin. Mutterlauge I wurde eingeengt, dann mit 60 ml Methanol versetzt und 24 Stunden zum Rückfluss erhitzt. Danach wurde das Gemisch auf 0 ° C gekühlt und genutscht (Nachwaschen mit wenig Methanol). Der Nutschkuchen wurde 16 Stunden am Hochvakuum bei Raumtemperatur getrocknet, wobei weitere 136 mg (all-E)-R,R′-Zeaxanthin erhalten wurden. Gesamtausbeute an isoliertem Zeaxanthin: 636 mg kristallines (all-E)-R,R′-Zeaxanthin mit Smp. 191-193 ° C. Das Filtrat (Mutterlauge II) wurde eingeengt und mit 20 ml Methylenchlorid digeriert. Der unlösliche Rückstand wurde abgenutscht. Einengen des Filtrates ergab 3,1 g rotes Oel, welches nicht aufgearbeitet wurde.

Beispiel 7

a) Eine Lösung von 24,6 g 5-[4(R)-Acetoxy-2,6,6-trimethyl-1-cyclohexenyl] -3-methyl-2E,4E-pentadienylacetat (Reinhet ca. 88%) in 150 ml 2-Propanol wurde bei 50 °C mit einer Suspension von 100 g 62-prozentigem p-Chlorbenzolsulfinsäure-Nattriumsalz in 200 ml Wasser versetzt und das Gemisch 24 Stunden bei 80 °C gerührt. Zur Aufarbeitung wurde das Reaktionsgemisch bei Raumtemperatur auf ein Gemisch von 80 ml Methylenchlorid und 320 ml Hexan gegossen. Die organische Phase wurde abgetrennt und dreimal mit je 200 ml Wasser gewaschen. Die wässrigen Phasen wurden mit 200 ml Methylenchlorid/Hexan (Vol. 1:4) nachextrahiert. Die organischen Phasen wurden vereinigt, über Natriumsulfat getrocknet, filtriert und eingeengt. Hierbei wurden 27,4 g Rohprodukt als orange-braunes Oel erhalten, welches an Kieselget mit Hexan/tert.Butylmethyläther chromatographisch getrennt wurde. Die reinen Fraktionen wurden am Rotationsverdampfer bis zur beginnenden Kristallisation eingeengt, dann im Kühlschrank auskristallisieren gelassen und genutscht (Nachwaschen mit kaltem Hexan). Der Nutschkuchen wurde 16 Stunden am Hochvakuum bei Raumtemperatur getrocknet, wobei 13,4 g 5-[4(R)-Acetoxy-2,6,6-trimethyl-1-cyclohexenyl] -3-methyl-2E,4E-pentadienyl-(p-chlorphenyl)sulfon als weisses Pulver mit Smp. 101-102 °C erhalten wurden. Einengen der Mutterlauge ergab weitere 3,8 g Produkt mit ca. 10% cis-Anteil als halbkristalline Masse.

b) 5-[4(R)-Acetoxy-2,6,6-trimethyl-1-cyclohexenyl]-3-methyl-2E,4E-pentadienyl-(p-chlorphenyl)sulfon wurde in analoger Weise zu beispiel 6, Stufen b-d via (all-E)-3(R),12, 3′(R),12′-Tetraacetoxy-11,11′ -bis[(p-chlorphenyl)sulfonyl]-11,12,11′,12′ -tetrahydro-zeaxanthin und das Diacetat von (all-E)-R,R′-Zeaxanthin-11,11′-ylen-bis-[(p-chlorphenyl)-sulfon zu (all-E)-R,R′-Zeaxanthin umgesetzt.

Beispiel 8

a) 34 ml (400 mMol) 36-prozentiger Salzsäure wurden unter Rühren und Stickstoffbegasung bei 25-30 °C innert 15 Minuten tropfenweise mit einer Lösung von 24 g (100 mMol) 3-(3-Hydroxy-3-methyl-1E,4-pentadienyl)2,4,4-trimethyl -2-cyclohexen-1-on (Reinheit 97,6%) in 100 ml Methylenchlorid versetzt. Das Gemisch wurden noch 20 Minuten bei 25-30 °C gerührt und dann auf 100 ml gesättigte Kochsalzlösung gegossen. Die organische Phase wurde abgetrennt und mit 100 ml gesättigter Kochsalzlösung und mit 100 ml halbgesättigter Natriumhydrogencarbonat-Lösung gewaschen. Die wässrigen Phasen wurden zweimal mit je 100 ml Methylenchlorid nachextrahiert. Die organischen Phasen wurden vereinigt, über Natriumsulfat und wenig Kaliumcarbonat getrocknet, filtriert und auf ein Volumen von ca. 40 ml eingeengt. Nach Zugabe von 100 ml Dimethylformamid wurden das restliche Methylenchlorid aus dem Gemisch abdestilliert.

b) Die erhaltene Lösung von 3-(5-Chlor-3-methyl-1E,3E-pentadienyl)-2,4,4-trimethyl -2-cyclohexen-1-on in Dimethylformamid wurde mit 100 ml Dimethylformamid verdünnt und dann bei 10 °C mit 24,8 g (125 mMol) p-Chlorbenzolsulfinsäure-Natriumsulz versetzt. Das Reaktionsgemisch wurde 1 Stunde bei 70 °C gerührt und dann auf 2 l Wasser gegossen und mit 350 ml Methylenchlorid versetzt. Die organische Phase wurden abgetrennt und zweimal mit je 1 l hablgesättigter Natriumhydrogencarbonat-Lösung und einmal mit 1 l Wasser gewaschen. Die wässrigen Phasen wurden mit 250 ml Methylenchlorid nachextrahiert. Die organischen Phasen wurden über Natriumsulfat getrocknet, filtriert und eingeengt. Das erhaltene beige Pulver (39 g) wurde in Methylenchlorid gelöst und durch Zugabe von Diisopropyläther und Abdestillieren des Methylenchlorids umkristallisiert. Die Kristalle wurden abfiltriert und 16 Stunden am Wasserstrahlvakuum bei 40 °C getrocknet. Ausbeute: 22,6 g 5-(2,6,6-Trimethyl-3-oxo-1-cyclohexenyl)-3-methyl -2E,4E-pentadienyl-(p-chlor-phenyl)sulfon.

c) Ein Gemisch von 1,2 g (3 mMol) 5-(2,6,6-Trimethyl)3-oxo-1-cyclohexenyl)3-3-methyl 2E,4E-pentadienyl-(p-chlorphenyl)sulfon, 2 ml (12 mMol) Orthoameisensäure-triäthylester, 0,67 ml (12 mMol) Aethylenglykol und 3 mg p-Toluolsulfonsäure wurde 2 Stunden bei 30 °C gerührt. Danach wurde das Gemisch mit 2 ml (12 mMol) Orthoameisensäure-triäthylester, 0,67 ml (12 mMol) Aethylenglykol und 3 mg p-Toluolsulfonsäure versetzt und weitere 2 Stunden gerührt. Anschliessend wurde das reaktionsgemisch mit 30 mg Natriumhydrogencarbonat versetzt und mit Diäthyläther/Methylenchlorid extrahiert. Die organische Phase wurde mit halbgesättigter Kochsalzlösung und mit Wasser gewaschen. Die Waschlösungen wurden mit Diäthyläther nachextrahiert. Die organischen Phasen wurden über Natriumsulfat getrocknet, filtriert und eingeengt. Das erhaltene gelbe Oel (2,26 g) wurde aus pentan kristallisiert. Die Kristalle wurden abgenutscht und 16 Stunden am Hockvakuum bei 40 °C getrocknet. Ausbeute: 1,13 g 5-(6,8,8-Trimethyl-1,4-dioxaspiro[4,5]dec-6-en-7-yl)-3-methyl -2E,4E-pentadienyl-(p-chlorphenyl)-sulfon als weisses Pulver mit Smp. 126-128 °C.

d) Eine Lösung von 1,05 g (2,4 mMol) 5-(6,8,8-Trimethyl-1,4-dioxaspiro[4,5]dec-6-en-7-yl)-3-methyl -2E,4E-pentadienyl-(p-chlorphenyl)sulfon in 8 ml Tetrahydrofuran wurde unter Stickstoffbegasung bei -60 °C tropfenweise zuerst innert 10 Minuten mit 1,5 ml einer 1,6M Lösung von Butyllithium (2,4 mMol) in Hexan und dann innert 10 Minuten mit einer Lösung von 164 mg (1 mMol) (all-E)-2,7-Dimethyl-2,4,6-octatriendial in 8 ml Tetrahydrofuran versetzt. Das Gemisch wurde noch 3,5 Stunden bei -60 °C gerührt, dann mit 1,4 ml Acetanhydrid versetzt und 2 Stunden bei 0 °C gerührt. Anschliessend wurde das Gemisch enthaltend (all-E)-12,12′-Diacetoxy-4,4,4′,4′-bis(äthylendioxy)-11,11′ -bis[(p-chlorphenyl)sulfonyl] -11,12,11′,12′-tetrahydro-canthaxanthin bei -5 °C bis +5 °C mit 6 ml 28-prozentiger wässriger Natronlauge versetzt und 1 Stunde im Eisbad und 16 Stunden bei Raumtemperatur gerührt. Danach wurde das orange Reaktionsgemisch mit Methylenchlorid extrahiert. Die organische Phase wurde mit gesättigter Natriumcarbonat-Lösung und mit 1-prozentiger Kochsalzlösung gewaschen, über Natriumsulfat getrocknet, filtriert und eingeengt. Das erhaltene orange Pulver (1,3 g) wurde aus Methylenchlorid/Diisopropyläther umkristallisiert. Ausbeute: 692 mg (all-E)-4,4,4′,4′-Bis(äthylendioxy)-canthaxanthin-11,11′-ylen -bis[(p-chlorphenyl)sulfon] als oranges Pulver.

e) Eine Suspension von 600 mg (0,6 mMol) (all-E)-4,4,4′,4′-Bis(äthylendioxy)-canthaxanthin-11,11′-ylen -bis[(p-chlorphenyl)sulfon in 60 ml 1,2-Dimethoxyäthan wurde mit 1,56 ml (15 mMol) Diäthylamin und mit einer Lösung von 1,05 g 85-prozentigem Natriumdithionit (5,1 mMol) in 15 ml Wasser versetzt. Das Gemisch wurde 3 Stunden bei 25-30 °C gerührt, dann auf 50 ml Eiswasser gegossen und mit Diäthyläther und Methylenchlorid versetzt. Die wässrige Phase wurde abgetrennt und zweimal mit je 50 ml Diäthyläther/Methylenchlorid nachextrahiert. Die oraganischen Phasen wurden zweimal mit je 50 ml 5-prozentiger Kaliumdihydrogenphosphat-Lösung gewaschen und dann mit 50 ml Wasser, 100 mg p-Toluolsulfonsäure und 5 ml Eisessig versetzt. Das Gemisch wurde 17 Stunden bei 30-35 °C gerührt. Anschliessend wurde die organische Phase abgetrennt, fünfmal mit je 50 ml 2-prozentiger Natriumhydrogencarbonat-Lösung gewaschen, über Natriumsulfat getrocknet, filtriert und am Rotationsverdampfer eingedampft. Das erhaltene dunkelviolette Pulver (341 mg Rohrprodukt) wurde mit Methylenchlorid und Methanol versetzt. Nach Abdestillieren des Methylenchlorids wurde das Gemisch noch 4,5 Stunden unter schwachem Rüchfluss gekocht und dann auf 0-5 °C gekühlt. Die Kristalle wurden abgenutscht und 16 Stunden im Hochvakuum bei 40 °C getrocknet. Ausbeute: 231 mg (all-E)-Canthaxanthin als feines Pulver mit Smp. 206-208 °C.

Beispiel 9

a) Ein Gemisch von 146 g 2-Hydroxy-2-methyl-3-butenal-dimethylacetal, 175 ml Methylenchlorid, 4,4 g Kupfer-(II)-chlorid-Dihydrat und 225 ml konzentrierter Salzsäure wurde unter Rühren 20 Stunden zum Rückfluss erwärmt. Anschliessend wurde das Reaktionsgemisch mit 25 ml Methylenchlorid in ein Ausrührgefäss gespült. Die organische Phase wurde abgetrennt und die wässrige Phase wurde viermal mit je 50 ml Methylenchlorid extrahiert. Die organischen Phasen wurden mit 25 ml 10-prozentiger Kochsalzlösung und mit 25 ml gesättigter Natriumhydrogencarbonat-Lösung gewaschen, über Natriumsulfat getrocknet, filtriert und eingedampft. Das erhaltene Rohprodukt wurde mit wenig Methylenchlorid in einen Kolben mit Hickmann-Aufsatz gespült. Anschliessend wurde zuerst am Wasserstrahlvakuum bei ca. 60 °C das Methylenchlorid abdestilliert und dann bei 45-55 °C/2 Torr das Produkt flach destilliert. Hierbei wurden 99 g γ-Chlortiglinaldehyd erhalten.

b) 67 g γ-Chlortiglinaldehyd wurden unter Stickstoffbegasung in 1 l Dimethylformamid gelöst. Die Lösung wurde auf 10 °C gekühlt und mit 260 g 62-prozentigem p-Chlorbenzolsulfinsäure-Natriumsalz versetzt. Das Gemisch wurde 1 Stunde bei 70 °C gerührt, dann auf Eis/Wasser gegossen und mit Methylenchlorid extrahiert. Die organische Phase wurde über Natriumsulfat getrocknet, filtriert und eingeengt. Kristallisation des erhaltenen braunes Oels aus Aethylacetat-Hexan ergab 85 g farblosen γ-[-(p-Chlorphenyl)sulfonyl]tiglinaldehyd mit Smp. 97-99 °C.

c) Eine Lösung von 10,4 g γ-[p-Chlorphenyl)sulfonly]tiglinadlehyd in 80 ml Methanol wurde unter Stickstoffbegasung mit 8,68 g Orthoameisensäuremethylester und 80 mg 85-prozentiger Phosphorsäure versetzt und über Nacht bei 30 °C gerührt. Anschliessend wurde das Reaktionsgemisch auf wässrige Natriumhydrogencarbonat-Lösung gegossen und mit Methylenchlorid extrahiert. Die organische Phase wurde über Natriumsulfat getrocknet, filtriert und eingeengt. Hierbei wurde quantitativ γ-[(p-Chlorphenyl)-sulfonly]tiglinaldehyd-dimethylacetal erhalten, welches spontan kristallisierte (Smp. 60-62 °C).

d)4,5 g γ-[(p-Chlorphenyl)sulfonyl]tiglinaldehyd-dimethylacetal wurden unter Inertgas in 22 ml Tetrahydrofuran gelöst. Die Lösung wurde auf -70 °C gekühlt und tropfenweise zuerst mit 9,11 ml einer 1,6M Lösung von Butyllithium in Hexan und dann innert 20 Minuten mit einer Lösung von 2,57 g 12′-Apo-β-carotin-12′-al in 30 ml Tetrahydrofuran versetzt. Das Reaktionsgemisch wurde über Nacht bei -70 °C

gerührt und dann innert 5 Minuten mit 5,6 ml Acetanhydrid versetzt. Das Gemisch wurde 6 Stunden bei ca. 3 °C (Kühlung mit einem Eisbad) gerührt, dann mit 26 ml 28-prozentiger wässriger Natronlauge versetzt und über Nacht bei raumtemperatur gerührt. Danach wurde die organische Phase abgetrennt, mit Diäthyläther verdünnt, mit wässriger Natriumhydrogencarbonat-Lösung und mit Kochsalzlösung neutralgewaschen, über Natriumsulfat getrocknet, filtriert und eingeengt. Hierbei wurden 8 g rohes (all-E)-4-[(p-Chlorphenyl)sulfonyl]-2,6,11,15-tetramethyl-17-(2,2,6-trimethyl-1-cyclohexen-1-yl)-2,4,6,8,10,12,14,16-heptadecaoctaenal-dimethylacetal als rotes Oel erhalten.

e) Ein gemisch von 8 g rohem (all-E)-4-[(p-Chlorphenyl)sulfonyl]-2,6,11,15-tetramethyl-17-(2,2,6-trimethyl-1-cyclohexen-1-yl)-2,4,6,8,10,12,14,16-heptadecaoctaenal-dimethylacetal, 30 ml Tetrahydrofuran, 30 ml Wasser, 3 ml Diäthylamin und 6,5 g Natriumdithionit wurde 2 Stunden bei Raumtemperatur gerührt. Anschliessend wurde das Reaktionsgemisch mit verdünnter Schwefelsäure angesäuert (Hydrolyse der Acetalgruppe), dann auf wässrige Natriumhydrogencarbonat-Lösung gegossen und mit Diäthyläther verdünnt. Die organische Phase wurde neutral gewaschen, über Natriumsulfat getrocknet, filtriert und eingeengt. Hierbei wurden 4,1 g rotes, öliges Rohprodukt von 8'-Apo-β-carotin-8'-al erhalten. Nach Erhitzen zum Rückfluss mit Heptan (Isomerisierung), Säulenchromatographie und Kristallisation aus Pentan konnte reines Produkt (all-E-Anteil 97%) mit Smp. 126-128 °C erhalten werden.

Beispiel 10

a) Eine Lösung von 7,6 g γ-[(p-Chlorphenyl)sulfonyl]tiglinaldehyd-dimethylacetal in 100 ml Tetrahydrofuran wurde auf -70 °C gekühlt und innert 10 Minuten tropfenweise mit 15 ml einer 1,6M Lösung von Butyllithium in Hexan versetzt. Dann wurde das Reaktionsgemisch innert 10 Minuten mit einer Lösung von 1,78 g (all-E)-2,7-Dimethyl-2,4,6-octatriendial in 50 ml Tetrahydrofuran versetzt und über Nacht bei -70 °C gerührt. Anschliessend wurde das Reaktionsgemisch bei -70 °C tropfenweise mit 15 ml Acetanhydrid versetzt und unter ansteigender Temperatur (bis zu -20 °C) weitergerührt. Nach 4 Stunden wurde das Reaktionsgemisch bei -20 °C tropfenweise mit 66 ml 28-prozentiger Natronlauge versetzt und über Nacht weitergerührt, wobei die Temperatur auf Raumtemperatur steigen gelassen wurde. Danach wurde das Reaktionsgemisch auf ein Gemisch von Eis und verdünnter wässriger Kochsalzlösung gegossen und mit Diäthyläther verdünnt. Die organische Phase wurde über Natriumsulfat getrocknet, filtriert und eingeengt. Hierbei wurden 10 g rohes (all-E)-4,13-Bis-[(p-chlorphenyl)sulfonyl]-2,6,11,15-tetramethyl-2,4,6,8,10,12,14-hexadecaheptaendial-bis(dimethylacetal) als rotes Oel erhalten.

b) Ein Gemisch von 10 g rohem (all-E)-4,13-Bis-[(p-chlorphenyl)sulfonyl]-2,6,11,15-tetramethyl-2,4,6,8,10,12,14-hexadecaheptaendial-bis(dimethylacetal), 150 ml Tetrahydrofuran, 100 ml Wasser, 36 ml Diäthylamin und 24 g Natriumdithionit wurde 40 Minuten bei Raumtemperatur gerührt und dann mit verdünnter Schwefelsäure versetzt. Die organische Phase wurde mit Diäthyläther verdünnt, mit wässriger Natriumhydrogencarbonat-Lösung gewaschen, mit wässriger Kochsalzlösung neutral gewaschen, über Natriumsulfat getrocknet, filtriert und eingeengt. Hierbei wurden 4,8 g rotes Oel als Rohprodukt von Crocetindialdehyd erhalten. Filtration des Rohproduktes durch Kieselgel und Kristallisation aus Methylenchlorid/Methanol ergab kristallinen Crocetindialdehyd mit Smp. 193-195 °C und einem all-E-Anteil von 98%. Die obige Mutterlauge (2,1 g) mit einem Gehlat von 78% Crocetindialdehyd wurde nicht aufgearbeitet.

## Patentansprüche

**1.** Verbindungen der allgemeinen Formel

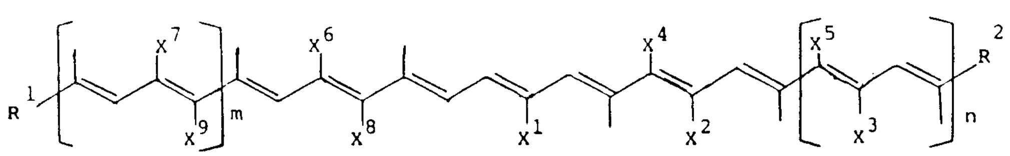

I

worin m die Zahl 1 und $R^1$ 4-Methyl-3-pentenyl bezeichnet, oder m die Zahl 0 und $R^1$ eine acetalisierte Formylgruppe, eine gegebenenfalls veresterte Carboxygruppe oder eine Gruppe der allgemeinen Formel

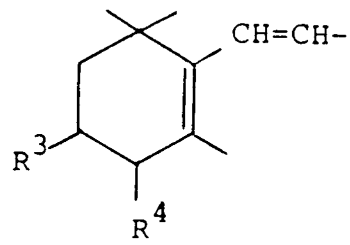

II

bezeichnet; n die Zahl 1 und $R^2$ 4-Methyl-3-pentenyl bezeichnet, oder n die Zahl 0 und $R^2$ eine acetalisierte Formylgruppe, eine gegebenenfalls veresterte Carboxygruppe oder eine Gruppe der Formel II bezeichnet; $R^3$ und $R^4$ unabhängig voneinander Wasserstoff, eine gegebenenfalls geschützte Hydroxygruppe oder eine geschützte Oxogruppe darstellen; eine der vorhandenen Gruppe $X^1$, $X^2$, $X^3$, $X^4$ und $X^5$ einen Sulfonylrest bedeutet und die übrigen Wasserstoff bedeuten; und $X^5$, $X^7$, $X^5$ und $X^5$ Wasserstoff bedeuten oder, wenn $R^1$ und $R^2$ gleiche Bedeutungen haben, auch $X^5$ die Bedeutung von $X^2$, $X^7$ die Bedeutung von $X^3$, $X^5$ die Bedeutung von $X^4$ und $X^5$ die Bedeutung von $X^5$ hat.

2. Verbindungen nach Anspruch 1 in der all-E-Form.

3. Verbindungen nach Anspruch 1 oder 2, dadurch gekennzeichnet, dass $X^1$, $X^2$ oder $X^3$ einen Sulfonylrest bedeutet.

4. Verbindungen nach Anspruch 1 oder 2, dadurch gekennzeichnet, dass $X^2$ einen Sulfonylrest, $X^5$ Wasserstoff oder einen Sulfonylrest und $X^1$, $X^3$, $X^4$, $X^5$, $X^7$, $X^5$ und $X^5$ Wasserstoff bedeuten oder $X^4$ einen Sulfonylrest, $X^5$ Wasserstoff oder einen Sulfonylrest und $X^1$, $X^2$, $X^3$, $X^5$, $X^5$, $X^7$ und $X^5$ Wasserstoff bedeuten.

5. Verbindungen nach Anspruch 1 oder 2, dadurch gekennzeichnet, dass m und n für die Zahl 1 stehen, $R^1$ und $R^2$ 4-Methyl-3-pentenyl bezeichnen, entweder die beiden Gruppen $X^3$ und $X^7$ oder die beiden Gruppen $X^5$ und $X^5$ Sulfonylreste darstellen und die übrigen der Gruppen $X^1$-$X^5$ jeweils Wasserstoff bedeuten.

6. Verbindungen nach einem der Ansprüche 1 bis 4, dadurch gekennzeichnet, dass $R^1$ und $R^2$ gleiche Bedeutung haben oder $R^2$ eine acetalisierte Formylgruppe bedeutet.

7. Verbindungen nach einem der Ansprüche 1 bis 4, dadurch gekennzeichnet, dass $R^3$ Wasserstoff oder eine gegbenenfalls geschützte Hydroxygruppe und $R^4$ Wasserstoff oder eine geschützte Oxogruppe bedeuten.

8. Verbindungen nach einem der Ansprüche 1 bis 7, dadurch gekennzeichnet, dass der Ausdruck Sulfonylrest für substituiertes oder unsubstituiertes Phenylsulfonyl steht.

9. Verbindungen der allgemeinen Formel

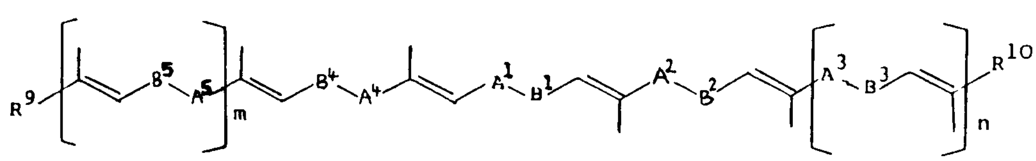

X

worin eine der Gruppen $-A^1-B^1-$, $-A^2-B^2-$ und $-A^3-B^3-$ die Gruppe -CHX-CH(OR)- oder -CH(OR)-CHX-darstellt und die beiden andern -CH=CH- darstellen; X einen Sulfonylrest und -OR Acyloxy bezeichnen; $-A^4-B^4-$ und $-A^5-B^5-$ für -CH=CH- stehen oder, wenn $R^{10}$ die Bedeutung von $R^9$ und n die Bedeutung von m hat, auch $-A^4-B^4-$ die Bedeutung von $-A^2-B^2-$ und $-A^5-B^5-$ die Bedeutung von $-A^3-B^3-$ hat; m die Zahl 1 und $R^9$ 4-Methyl-3-pentenyl bezeichnet, oder m die Zahl 0 und $R^9$ eine acetalisierte Formylgruppe, eine gegebenenfalls veresterte Carboxygruppe oder eine Gruppe der allgemeinen

Formel

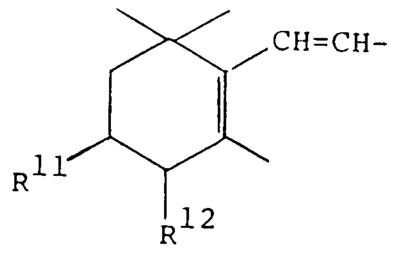

IX

bezeichnet; n die Zahl 1 und $R^{10}$ 4-Methyl-3-pentenyl bezeichnet, oder n die Zahl 0 und $R^{10}$ eine acetalisierte Formylgruppe, eine gegebenenfalls veresterte Carboxygruppe oder eine Gruppe der Formel IX bezeichnet; und $R^{11}$ und $R^{12}$ unabhängig voneinander Wasserstoff, eine gegebenenfalls geschützte Hydroxygruppe oder eine geschützte Oxogruppe darstellen.

**10.** Verbindungen nach Anspruch 9 in der all-trans-Form.

**11.** Verfahren zur Herstellung der Verbindungen der allgemeinen Formel

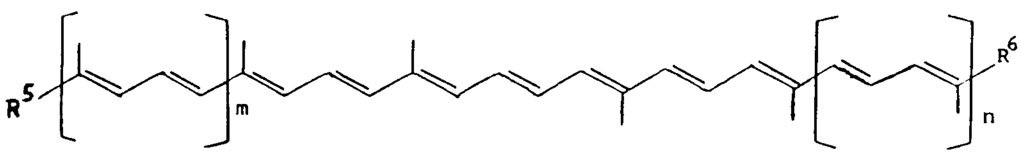

III

worin m die Zahl 1 und $R^5$ 4-Methyl-3-pentenyl bezeichnet, oder m die Zahl 0 und $R^5$ eine gegebenenfalls acetalisierte Formylgruppe, eine gegebenenfalls veresterte Carboxygruppe oder eine Gruppe der allgemeinen Formel

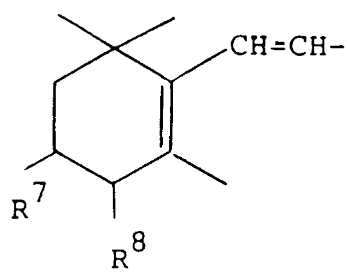

IV

bezeichnet; n die Zahl 1 und $R^6$ 4-Methyl-3-pentenyl bezeichnet, oder n die Zahl 0 und $R^6$ eine gegebenenfalls acetalisierte Formylgruppe, eine gegebenenfalls veresterte Carboxygruppe oder eine Gruppe der Formel IV bezeichnet; und $R^7$ und $R^8$ unabhängig voneinander Wasserstoff, eine gegebenenfalls geschützte Hydroxygruppe oder eine gegebenenfalls geschützte Oxogruppe darstellen.
dadurch gekennzeichnet, dass man eine Verbindung der allgemeinen Formel

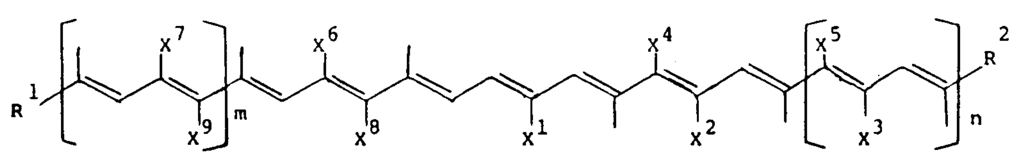

I

worin m die Zahl 1 und $R^1$ 4-Methyl-3-pentenyl bezeichnet, oder m die Zahl 0 und $R^1$ eine acetalisierte Formylgruppe, eine gegebenenfalls veresterte Carboxygruppe oder eine Gruppe der

allgemeinen Formel

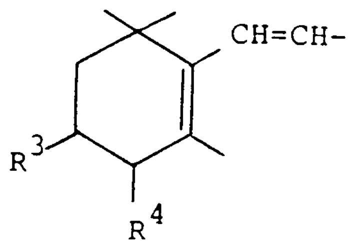

II

bezeichnet; n die Zahl 1 und $R^2$ 4-Methyl-3-pentenyl bezeichnet, oder n die Zahl 0 und $R^2$ eine acetalisierte Formylgruppe, eine gegebenenfalls veresterte Carboxygruppe oder eine Gruppe der Formel II bezeichnet; $R^3$ und $R^4$ unabhängig voneinander Wasserstoff, eine gegebenenfalls geschützte Hydroxygruppe oder eine geschützte Oxogruppe darstellen; eine der vorhandenen Gruppen $X^1$, $X^2$, $X^3$, $X^4$ und $X^5$ einen Sulfonylrest bedeutet und die übrigen Wasserstoff bedeuten; und $X^5$, $X^7$, $X^5$ und $X^5$ Wasserstoff bedeuten oder, wenn $R^1$ und $R^2$ gleiche Bedeutungen haben, auch $X^5$ die Bedeutung von $X^2$, $X^7$ die Bedeutung von $X^3$, $X^5$ die Bedeutung von $X^4$ und $X^5$ die Bedeutung von $X^5$ hat.
mit Dithionit in einem wässrig-organischen Lösungsmittelgemisch in Gegenwart von Ammoniak oder einem aliphatischen Amin reduziert und dass man, gewünschtenfalls, vorhandene Schutzgruppen abspaltet.

**12.** Verfahren nach Anspruch 11, dadurch gekennzeichnet, dass man die Reduktion mit Alkalimetalldithionit, vorzugsweise mit Natriumdithionit durchführt.

**13.** Verfahren nach Anspruch 11 oder 12, dadurch gekennzeichnet, dass man die Reduktion in Gegenwart von Ammoniak oder in Gegenwart eines aliphatischen Amins durchführt, welches einen pKa-Wert von mindesterns 9,25, gemessen in wässriger Lösung bei 20 °C besitzt.

**14.** Verfahren nach einem der Ansprüche 11 bis 13, dadurch gekennzeichnet, dass man die Reduktion in Gegenwart von Ammoniak, Alkylamin, Dialkylamin, Trialkylamin, Alkylendiamin oder einem cyclischen aliphatischen Amin durchführt.

**15.** Verfahren nach einem der Ansprüche 11 bis 14, dadurch gekennzeichnet, dass man die Reduktion in Gegenwart eines Ueberschusses an Ammoniak oder aliphatischem Amin durchführt.

**16.** Verfahren nach einem der Ansprüche 11 bis 15, dadurch gekennzeichnet, dass man die Reduktion in einem Wasser/Aether-Gemisch, vorzugsweise in einem Gemisch von Wasser und Tetrahydrofuran oder 1,2-Dimethoxyäthan durchführt.

**17.** Verfahren nach einem der Ansprüche 11 bis 16, dadurch gekennzeichnet, dass man eine all-E-Verbindung der Formel I umsetzt.

**18.** Verfahren zur Herstellung der Verbindungen der allgemeinen Formel

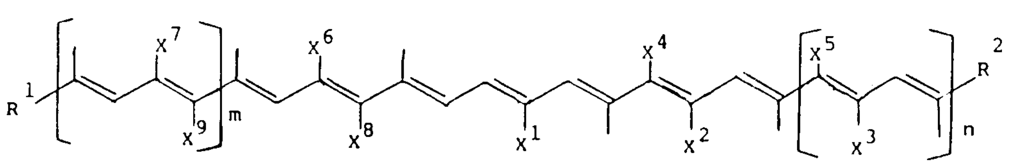

I

worin m die Zahl 1 und $R^1$ 4-Methyl-3-pentenyl bezeichnet, oder m die Zahl 0 und $R^1$ eine acetalisierte Formylgruppe, eine gegebenenfalls veresterte Carboxygruppe oder eine Gruppe der algemeinen Formel

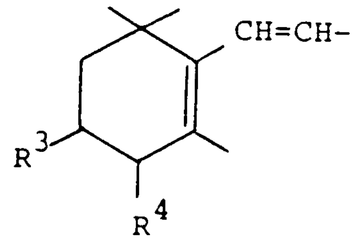

II

bezeichnet; n die Zahl 1 und $R^2$ 4-Methyl-3-pentenyl bezeichnet, oder n die Zahl 0 und $R^2$ eine acetalisierte Formylgruppe, eine gegebenenfalls veresterte Carboxygruppe oder eine Gruppe der Formel II bezeichnet; $R^3$ und $R^4$ unabhängig voneinander Wasserstoff, eine gegebenenfalls geschützte Hydroxygruppe oder eine geschützte Oxogruppe darstellen; eine der vorhandenen Gruppen $X^1$, $X^2$, $X^3$, $X^4$ und $X^5$ einen Sulfonylrest bedeutet und die übrigen Wasserstoff bedeuten; und $X^5$, $X^7$, $X^5$ und $X^5$ Wasserstoff bedeuten oder, wenn $R^1$ und $R^2$ gleiche Bedeutungen haben, auch $X^5$ die Bedeutung von $X^2$, $X^7$ die Bedeutung von $X^3$, $X^5$ die Bedeutung von $X^4$ und $X^5$ die Bedeutung von $X^5$ hat.

dadurch gekennzeichnet, dass man

a) eine Verbindung der allgemeinen Formel

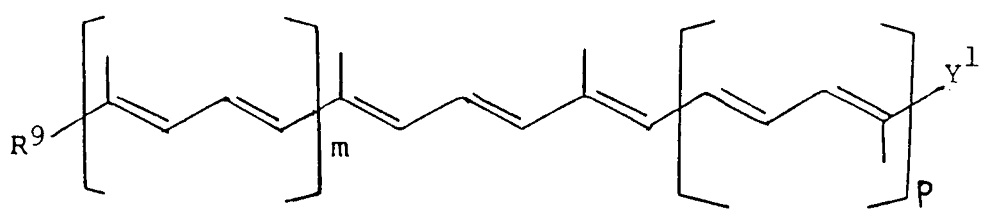

V

mit einer Verbindung der allgemeinen Formel

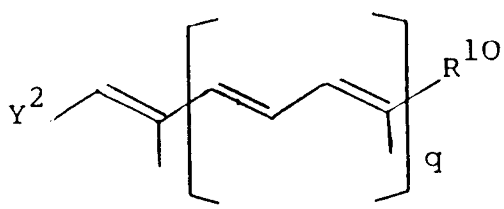

VI

oder

b) eine Verbindung der allgemeinen Formel

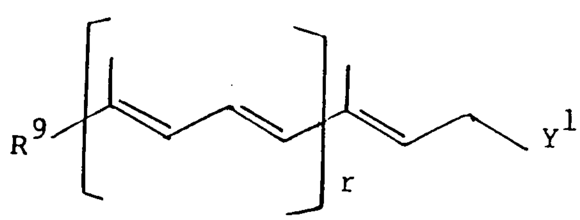

VII

mit einer Verbindung der allgemeinen Formel

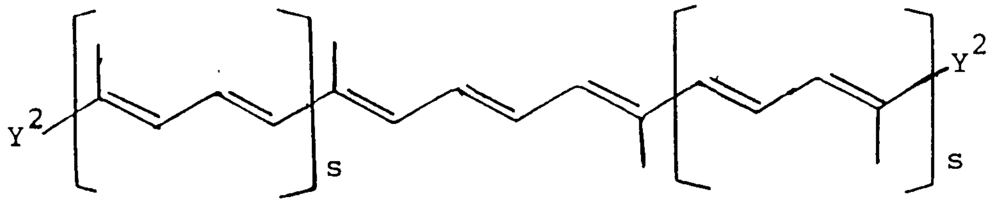

VIII

worin eine der Gruppen $Y^1$ und $Y^2$ Formyl und die andere eine Gruppe $-CH_2X$ darstellt; X einen Sulfonylrest darstellt; p und q für die Zahlen 0, 1 oder 2 stehen und $p + q = n + 1$ beträgt; r und s für die Zahlen 0 oder 1 stehen und $r + s = m$ beträgt, m die Zahl 1 und $R^9$ 4-Methyl-3-pentenyl bezeichnet, oder m die Zahl 0 und $R^9$ eine acetalisierte Formylgruppe, eine gegebenenfalls veresterte Carboxygruppe oder eine Gruppe der allgemeinen Formel

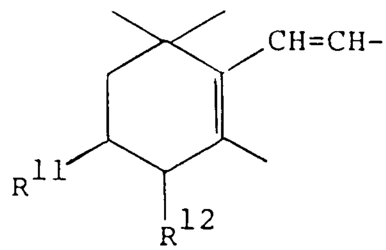

IX

bezeichnet; n die Zahl 1 und $R^{10}$ 4-Methyl-3-pentenyl bezeichnet, oder n die Zahl 0 und $R^{10}$ eine acetalisierte Formylgruppe, eine gegebenenfalls veresterte Carboxygruppe oder eine Gruppe der Formel IX bezeichnet; und $R^{11}$ und $R^{12}$ unabhängig voneinander Wasserstoff, eine gegebenenfalls geschützte Hydroxygruppe oder eine geschützte Oxogruppe darstellen,

in einem inerten organischen Lösungsmittel in Gegenwart einer Base umsetzt und die nach Zugabe eines Acylierungsmittels erhaltene Verbindung der allgemeinen Formel

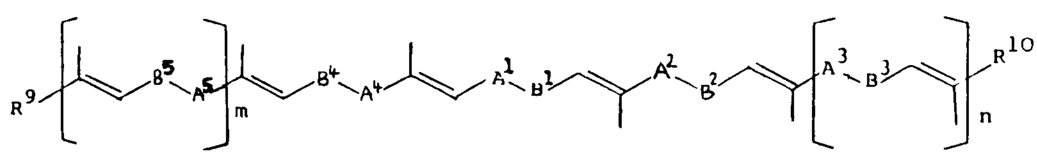

X

worin eine der Gruppen $-A^1-B^1-$, $-A^2-B^2-$ und $-A^3-B^3-$ die Gruppe -CHX-CH(OR)- oder -CH(OR)-CHX- darstellt und die beiden andern -CH=CH- darstellen; $-A^4-B^4-$ und $-A^5-B^5-$ für -CH=CH- stehen oder, wenn $R^{10}$ die Bedeutung von $R^9$ und n die Bedeutung von m hat, auch $-A^4-B^4-$ die Bedeutung von $-A^2-B^2-$ und $-A^5-B^5-$ die Bedeutung von $-A^3-B^3-$ hat; X einen Sulfonylrest darstellt; -OR Acyloxy bezeichnet; $R^9$, $R^{10}$, m und n die obigen Bedeutungen haben; und $R^{11}$ und $R^{12}$ unabhängig voneinander Wasserstoff, eine geschützte Hydroxygruppe oder eine geschützte Oxogruppe darstellen,

mit wässriger Base in eine Verbindung der Formel I überführt.

**19.** Verfahren nach Anspruch 18, dadurch gekennzeichnet, dass man als wässrige Base Alkalimetallhydroxid oder Erdalkalimetallhydroxid in Wasser, vorzugsweise Natronlauge oder Kalilauge verwendet.

**20.** Verfahren nach Anspruch 18 oder 19, dadurch gekennzeichnet, dass man die trans-Isomere bzw. die all-trans-Isomere der Verbindungen der Formeln V und VI oder der Verbindungen der Formeln VII und VIII umsetzt.

## Claims

1. Compounds of the general formula

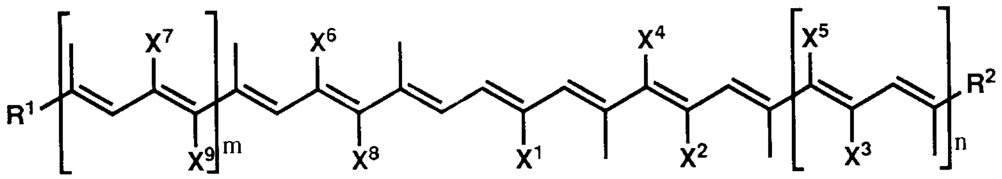

I

wherein m denotes the number 1 and $R^1$ denotes 4-methyl-3-pentenyl or m denotes the number 0 and $R^1$ denotes an acetalized formyl group, an optionally esterified carboxy group or a group of the general formula

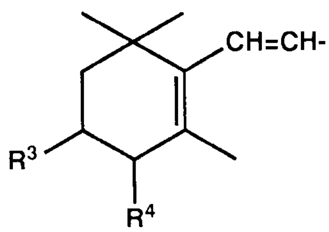

II

n denotes the number 1 and $R^2$ denotes 4-methyl-3-pentenyl or n denotes the number 0 and $R^2$ denotes an acetalized formyl group, an optionally esterified carboxy group or a group of formula II; $R^3$ and $R^4$ each independently represent hydrogen, an optionally protected hydroxy group or a protected oxo group; one of the groups $X^1$, $X^2$, $X^3$, $X^4$ and $X^5$ signifies a sulphonyl residue and the remainder signify hydrogen; and $X^5$, $X^7$, $X^5$ and $X^5$ signify hydrogen or, when $R^1$ and $R^2$ have the same significances, also $R^6$ has the significance of $X^2$, $X^7$ has the significance of $X^3$, $X^5$ has the significance of $X^4$ and $X^5$ has the significance of $X^5$.

2. Compounds according to claim 1 in the all-E form.

3. Compounds according to claim 1 or 2, characterized in that $X^1$, $X^2$ or $X^3$ signifies a sulphonyl residue.

4. Compounds according to claim 1 or 2, characterized in that $X^2$ signifies a sulphonyl residue, $X^5$ signifies hydrogen or a sulphonyl residue and $X^1$, $X^3$, $X^4$, $X^5$, $X^7$, $X^5$ and $X^5$ signify hydrogen or $X^4$ signifies a sulphonyl residue, $X^5$ signifies hydrogen or a sulphonyl residue and $X^1$, $X^2$, $X^3$, $X^5$, $X^5$, $X^7$ and $X^5$ signify hydrogen.

5. Compounds according to claim 1 or 2, characterized in that m and n stand for the number 1, $R^1$ and $R^2$ denote 4-methyl-3-pentenyl, either the two groups $X^3$ and $X^7$ or the two groups $X^5$ and $X^5$ represent sulphonyl residues and the remainder of the groups $X^1$-$X^5$ each signify hydrogen.

6. Compounds according to any one of claims 1 to 4, characterized in that $R^1$ and $R^2$ have the same significance or $R^2$ signifies an acetalized formyl group.

7. Compounds according to any one of claims 1 to 4, characterized in that $R^3$ signifies hydrogen or an optionally protected hydroxy group and $R^4$ signifies hydrogen or a protected oxo group.

8. Compounds according to any one of claims 1 to 7, characterized in that the term sulphonyl residue stands for substituted or unsubstituted phenylsulphonyl.

**9.** Compounds of the general formula

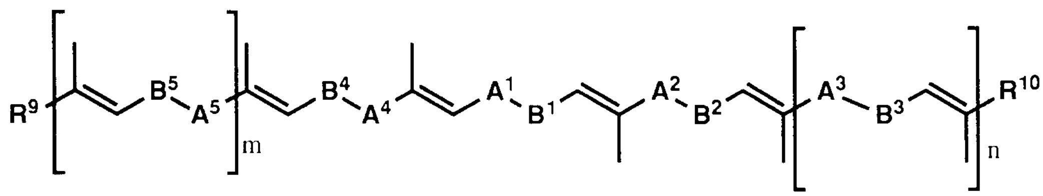

X

wherein one of the groups $-A^1B^1-$, $-A^2-B^2-$ and $-A^3-B^3-$represents the group -CHX-CH(OR)- or -CH-(OR)-CHX- and the other two represent -CH=CH-; X denotes a sulphonyl residue and -OR denotes acyloxy; $-A^4-B^4-$ and $-A^5-B^5-$ stand for -CH=CH- or, when $R^{10}$ has the significance of $R^9$ and n has the significance of m, also $-A^4-B^4-$ has the significance of $-A^2-B^2-$ and $-A^5-B^5-$ has the significance of $-A^3-B^3-$; m denotes the number 1 and $R^9$ denotes 4-methyl-3-pentenyl or m denotes the number 0 and $R^9$ denotes an acetalized formyl group, an optionally esterified carboxy group or a group of the general formula

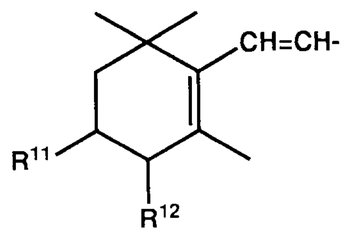

IX

n denotes the number 1 and $R^{10}$ denotes 4-methyl-3-pentenyl or n denotes the number 0 and $R^{10}$ denotes an acetalized formyl group, an optionally esterified carboxy group or a group of formula IX; and $R^{11}$ and $R^{12}$ each independently represent hydrogen, an optionally protected hydroxy group or a protected oxo group.

**10.** Compounds according to claim 9 in the all-trans form.

**11.** A process for the manufacture of the compounds of the general formula

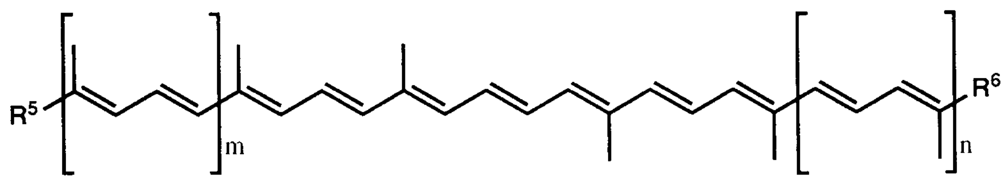

III

wherein m denotes the number 1 and $R^5$ denotes 4-methyl-3-pentenyl or m denotes the number 0 and $R^5$ denotes an optionally acetalized formyl group, an optionally esterified carboxy group or a group of the general formula

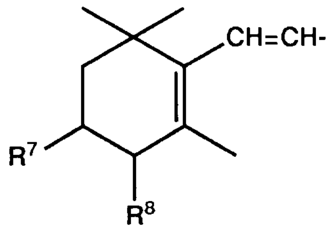

IV

n denotes the number 1 and $R^6$ denotes 4-methyl-3-pentenyl or n denotes the number 0 and $R^6$ denotes an optionally acetalized formyl group, an optionally esterified carboxy group or a group of formula IV; and $R^7$ and $R^8$ each independently represent hydrogen, an optionally protected hydroxy group or an optionally protected oxo group,
characterized by reducing a compound of the general formula

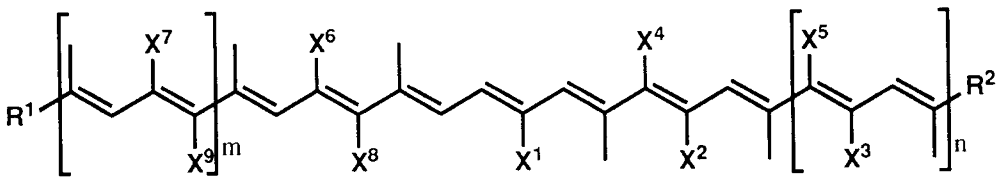

I

wherein m denotes the number 1 and $R^1$ denotes 4-methyl-3-pentenyl or m denotes the number 0 and $R^1$ denotes an acetalized formyl group, an optionally esterified carboxy group or a group of the general formula

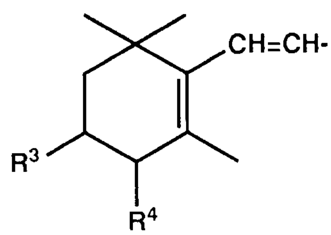

II

n denotes the number 1 and $R^2$ denotes 4-methyl-3-pentenyl or n denotes the number 0 and $R^2$ denotes an acetalized formyl group, an optionally esterified carboxy group or a group of formula II; $R^3$ and $R^4$ each independently represent hydrogen, an optionally protected hydroxy group or a protected oxo group; one of the groups $X^1$, $X^2$, $X^3$, $X^4$ and $X^5$ signifies a sulphonyl residue and the remainder signify hydrogen; and $X^5$, $X^7$, $X^5$ and $X^5$ signify hydrogen or, when $R^1$ and $R^2$ have the same significances, also $R^6$ has the significance of $X^2$, $X^7$ has the significance of $X^3$, $X^5$ has the significance of $X^4$ and $X^5$ has the significance of $X^5$,
with dithionite in an aqueous-organic solvent mixture in the presence of ammonia or an aliphatic amine and, if desired, cleaving off protecting groups present.

**12.** A process according to claim 11, characterized in that the reduction is carried out with alkali metal dithionite, preferably with sodium dithionite.

**13.** A process according claim 11 or 12, characterized in that the reduction is carried out in the presence of ammonia or in the presence of an aliphatic amine which has a pKa value of at least 9.25 measured in aqueous solution at 20 ° C.

**14.** A process according to any one of claims 11 to 13, characterized in that the reduction is carried out in the presence of ammonia or an alkylamine, dialkylamine, trialkylamine, alkylenediamine or cyclic aliphatic amine.

**15.** A process according to any one of claims 11 to 14, characterized in that the reduction is carried out in the presence of an excess of ammonia or aliphatic amine.

**16.** A process according to any one of claims 11 to 15, characterized in that the reduction is carried out in a water/ether mixture, preferably in a mixture of water and tetrahydrofuran or 1,2-dimethoxyethane.

**17.** A process according to any one of claims 11 to 16, characterized in that an all-E compound of formula I is reacted.

**18.** A process for the preparation of the compounds of the general formula

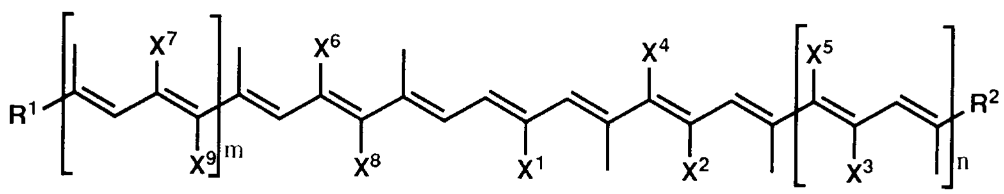

I

wherein m denotes the number 1 and $R^1$ denotes 4-methyl-3-pentenyl or m denotes the number 0 and $R^1$ denotes an acetalized formyl group, an optionally esterified carboxy group or a group of the general formula

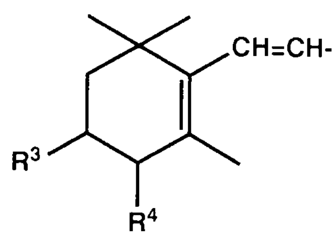

II

n denotes the number 1 and $R^2$ denotes 4-methyl-3-pentenyl or n denotes the number 0 and $R^2$ denotes an acetalized formyl group, an optionally esterified carboxy group or a group of formula II; $R^3$ and $R^4$ each independently represent hydrogen, an optionally protected hydroxy group or a protected oxo group; one of the groups $X^1$, $X^2$, $X^3$, $X^4$ and $X^5$ signifies a sulphonyl residue and the remainder signify hydrogen; and $X^5$, $X^7$, $X^5$ and $X^5$ signify hydrogen or, when $R^1$ and $R^2$ have the same significances, also $R^6$ has the significance of $X^2$, $X^7$ has the significance of $X^3$, $X^5$ has the significance of $X^4$ and $X^5$ has the significance of $X^5$,
characterized by
a) reacting a compound of the general formula

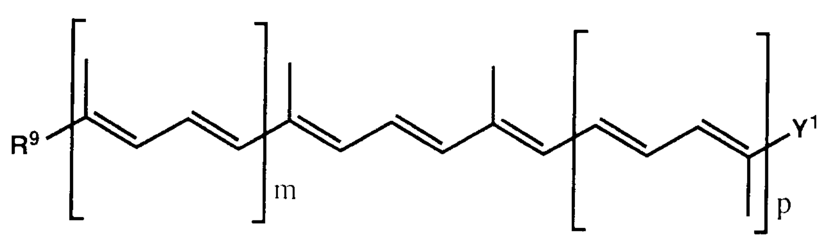

V

with a compound of the general formula

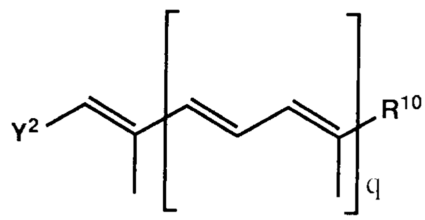

VI

or

b) reacting a compound of the general formula

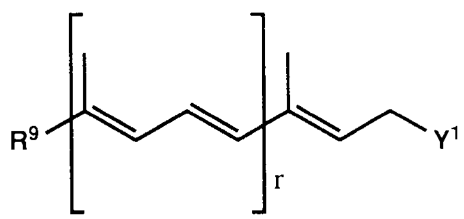

VII

with a compound of the general formula

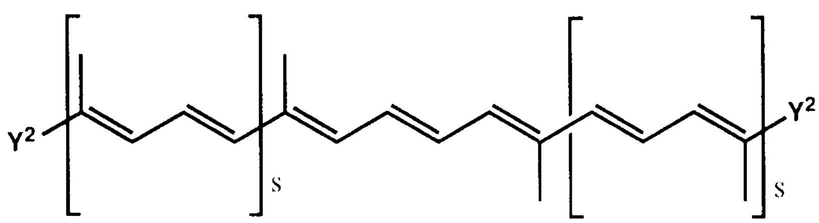

VIII

wherein one of the groups $Y^1$ and $Y^2$ represents formyl and the other represents a group $-CH_2X$; X represents a sulphonyl residue; p and q stand for the numbers 0,1 or 2 and $p + q = n + 1$; r and s stand for the numbers 0 or 1 and $r + s = m$; m denotes the number 1 and $R^9$ denotes 4-methyl-3-pentenyl or m denotes the number 0 and $R^9$ denotes an acetalized formyl group, an optionally esterified carboxy group or a group of the general formula

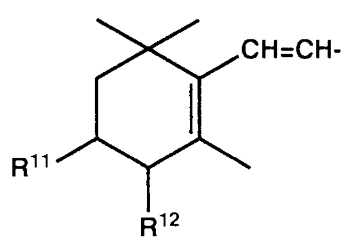

IX

n denotes the number 1 and $R^{10}$ denotes 4-methyl-3-pentenyl or n signifies the number 0 and $R^{10}$ denotes an acetalized formyl group, an optionally esterified carboxy group or a group of formula IX; and $R^{11}$ and $R^{12}$ each independently represent hydrogen, an optionally protected hydroxy group or a protected oxo group,

in an inert organic solvent in the presence of a base and, after the addition of an acylating agent, converting the resulting compound of the general formula

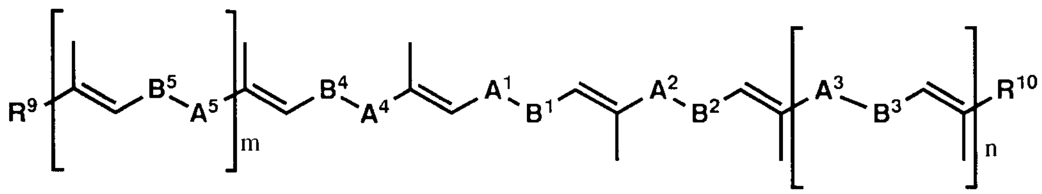

X

wherein one of the groups $-A^1-B^1-$, $-A^2-B^2-$ and $-A^3-B^3-$represents the group -CHX-CH(OR)- or -CH-(OR)-CHX- and the other two represent -CH=CH-; $-A^4-B^4-$ and $-A^5-B^5-$ stand for -CH=CH- or, when $R^{10}$ has the significance of $R^9$ and n has the significance of m, also $-A^4-B^4-$ has the significance of $-A^2-B^2-$ and $-A^5-B^5-$ has the significance of $-A^3-B^3-$; X represents a sulphonyl residue; -OR denotes acyloxy; $R^9$, $R^{10}$, m and n have the above significances; and $R^{11}$ and $R^{12}$ each independently represent hydrogen, a protected hydroxy group or a protected oxo group,
with aqueous base into a compound of formula I.

**19.** A process according to claim 18, characterized in that an alkali metal hydroxide or alkaline earth metal hydroxide in water, preferably sodium hydroxide solution or potassium hydroxide solution, is used as the aqueous base.

**20.** A process according to claim 18 or 19, characterized in that the trans isomers or the all-trans isomers of the compounds of formulae V and VI or of the compounds of formulae VII and VIII are reacted.

**Revendications**

**1.** Composés de formule générale

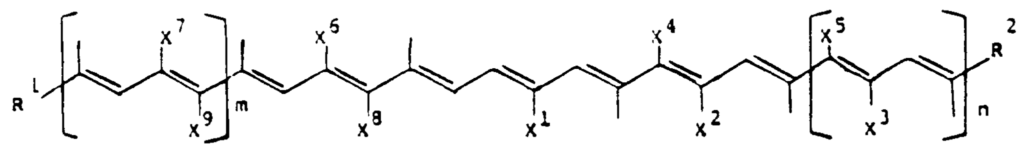

I

où m est égal à 1 et $R^1$ représente le 4-méthyl-3-pentényle ou m est égal à 0 et $R^1$ représente un groupe formyle acétalisé, un groupe carboxy éventuellement estérifié ou un groupe de formule générale

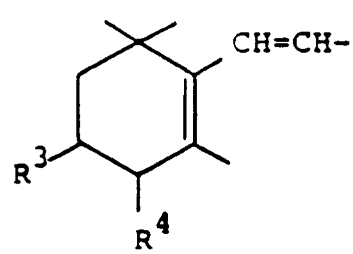

II

n est égal à 1 et $R^2$ représente le 4-méthyl-3-pentényle ou n est égal à 0 et $R^2$ représente un groupe formyle acétalisé, un groupe carboxy éventuellement estérifié ou un groupe de formule II ; $R^3$ et $R^4$ représentent, indépendamment l'un de l'autre, l'hydrogène, un groupe hydroxy éventuellement protégé ou un groupe oxo protégé ; l'un des groupes $X^1$, $X^2$, $X^3$, $X^4$ et $X^5$ présents représente un reste sulfonyle et les autres groupes l'hydrogène ; et $X^5$, $X^7$, $X^5$ et $X^5$ représentent l'hydrogène ou, lorsque $R^1$ et $R^2$ ont la même signification, également $X^5$ a la signification de $X^2$, $X^7$ a la signification de $X^3$, $X^5$ a la signification de $X^4$ et $X^5$ a la signification de $X^5$.

**2.** Composés selon la revendication 1 sous forme entièrement E.

**3.** Composés selon la revendication 1 ou 2, caractérisés en ce que $X^1$, $X^2$ ou $X^3$ représentent un reste sulfonyle.

**4.** Composés selon la revendication 1 ou 2, caractérisés en ce que $X^2$ représente un reste sulfonyle, $X^6$ l'hydrogène ou un reste sulfonyle et $X^1$, $X^3$, $X^4$, $X^6$, $X^7$, $X^6$ et $X^6$ représentent l'hydrogène ou $X^4$ représente un reste sulfonyle, $X^6$ représente l'hydrogène ou un reste sulfonyle et $X^1$, $X^2$, $X^3$, $X^6$, $X^6$, $X^7$ et $X^6$ représentent l'hydrogène.

**5.** Composés selon la revendication 1 ou 2, caractérisés en ce que m et n sont égaux à 1, $R^1$ et $R^2$ représentent le 4-méthyl-3-pentényle, les deux groupes $X^3$ et $X^7$ ou les deux groupes $X^6$ et $X^6$ représentent les restes sulfonyles et les autres groupes $X^1$-$X^6$ représentent l'hydrogène.

**6.** Composés selon l'une des revendications 1 à 4, caractérisés en ce que $R^1$ et $R^2$ ont la même signification ou $R^2$ représente un groupe formyle acétalisé.

**7.** Composés selon l'une des revendications 1 à 4, caractérisés en ce que $R^3$ représente l'hydrogène ou un groupe hydroxy éventuellement protégé et $R^4$ représente l'hydrogène ou un groupe oxo protégé.

**8.** Composés selon l'une des revendications 1 à 7, caractérisés en ce que l'expression reste sulfonyle signifie un phénylsulfonyle substitué ou non substitué.

**9.** Composés de formule générale

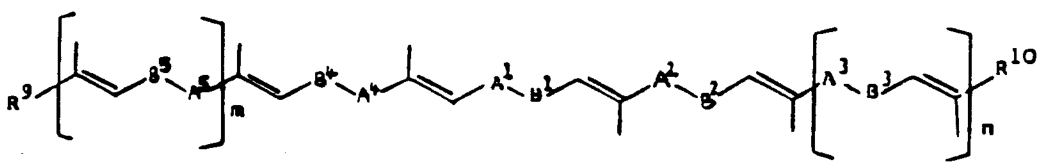

X

où l'un des groupes $-A^1-B^1-$, $-A^2-B^2-$ et $-A^3-B^3-$représente le groupe -CHX-CH(OR)- ou -CH(OR)-CHX- et les deux autres groupes -CH=CH- ; X désigne un reste sulfonyle et -OR un acyloxy ; $-A^4-B^4-$ et $-A^5-B^5-$représentent -CH=CH- ou, lorsque $R^{10}$ a la signification de $R^9$ et n a la signification de m, $-A^4-B^4-$ a également la signification de $-A^2-B^2-$ et $-A^5-B^5-$ a également la signification de $-A^3-B^3-$ ; m est égal à 1 et $R^9$ représente le 4-méthyl-3-pentényle ou m est égal à 0 et $R^9$ représente un groupe formyle acétalisé, un groupe carboxy éventuellement estérifié ou un groupe de formule générale

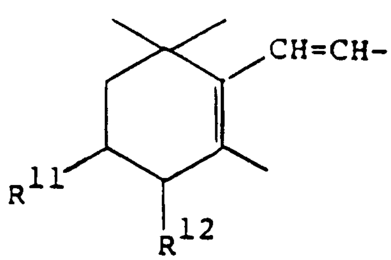

IX

n est égal à 1 et $R^{10}$ représente le 4-méthyl-3-pentényle ou n est égal à 0 et $R^{10}$ représente un groupe formyle acétalisé, un groupe carboxy éventuellement estérifié ou un groupe de formule IX : et $R^{11}$ et $R^{12}$ représentent, indépendamment l'un de l'autre, l'hydrogène, un groupe hydroxy éventuellement protégé ou un groupe oxo protégé.

**10.** Composés selon la revendication 9 sous forme entièrement trans.

**11.** Procédé pour la préparation des composés de formule générale

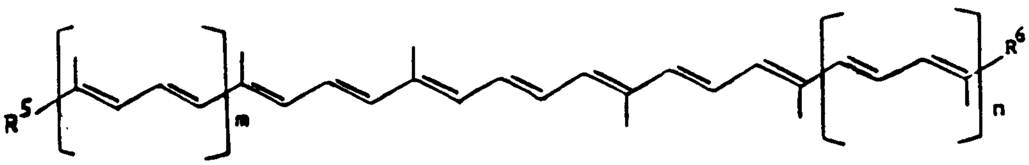

III

où m est égal à 1 et $R^5$ représente le 4-méthyl-3-pentényle ou m est égal à 0 et $R^5$ représente un groupe formyle éventuellement acétalisé, un groupe carboxy éventuellement estérifié ou un groupe de formule générale

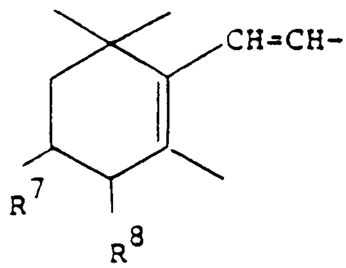

IV

n est égal à 1 et $R^6$ représente le 4-méthyl-3-pentényle ou n est égal à 0 et $R^6$ représente un groupe formyle éventuellement acétalisé, un groupe carboxy éventuellement estérifié ou un groupe de formule IV ; et $R^7$ et $R^8$ représentent, indépendamment l'un de l'autre, l'hydrogène, un groupe hydroxy éventuellement protégé ou un groupe oxo éventuellement protégé,
caractérisé en ce que l'on réduit un composé de formule générale

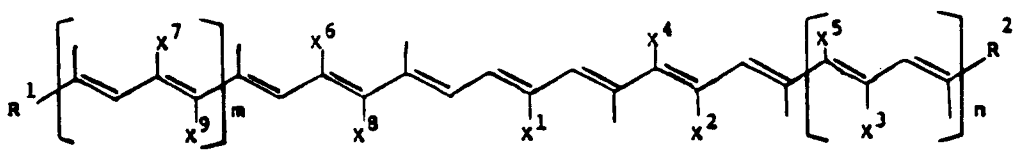

I

où m est égal à 1 et $R^1$ représente le 4-méthyl-3-pentényle ou m est égal à 0 et $R^1$ représente un groupe formyle acétalisé, un groupe carboxy éventuellement estérifié ou un groupe de formule générale

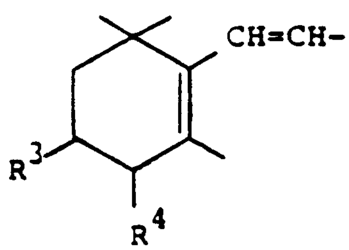

II

n est égal à 1 et $R^2$ représente le 4-méthyl-3-pentényle ou n est égal à 0 et $R^2$ représente un groupe formyle acétalisé, un groupe carboxy éventuellement estérifié ou un groupe de formule II ; $R^3$ et $R^4$ représentent, indépendamment l'un de l'autre, l'hydrogène, un groupe hydroxy éventuellement protégé ou un groupe oxo protégé ; l'un des groupes $X^1$, $X^2$, $X^3$, $X^4$ et $X^5$ présents représente un reste sulfonyle et les autres groupes l'hydrogène ; et $X^5$, $X^7$, $X^5$ et $X^5$ représentent l'hydrogène ou, lorsque $R^1$ et $R^2$ ont la même signification, également $X^5$ a la signification de $X^2$, $X^7$ a la signification de $X^3$, $X^5$ a la

signification de $X^4$ et $X^9$ a la signification de $X^9$, avec le dithionite dans un mélange de solvants hydroorganiques, en présence d'ammoniac ou d'une amine aliphatique et en ce que l'on clive, si désiré, les groupes protecteurs présents.

**12.** Procédé selon la revendication 11, caractérisé en ce que l'on effectue la réduction avec un dithionite de métal alcalin, de préférence, avec le dithionite de sodium.

**13.** Procédé selon la revendication 11 ou 12, caractérisé en ce que l'on effectue la réduction en présence d'ammoniac ou en présence d'une amine aliphatique, ayant un pKa d'au moins 9,25, mesuré dans une solution aqueuse à 20 ° C.

**14.** Procédé selon l'une des revendications 11 à 13, caractérisé en ce que l'on effectue la réduction, en présence d'ammoniac, d'une alkylamine, d'une dialkylamine, d'une trialkylamine, d'une alkylènediamine ou d'une amine cyclique aliphatique.

**15.** Procédé selon l'une des revendications 11 à 14, caractérisé en ce que l'on effectue la réduction en présence d'un excès d'ammoniac ou d'un excès d'une amine aliphatique.

**16.** Procédé selon l'une des revendications 11 à 15, caractérisé en ce que l'on effectue la réduction dans un mélange eau/éther, de préférence, dans un mélange d'eau et de tétrahydrofuranne ou de 1,2-diméthoxyéthane.

**17.** Procédé selon l'une des revendications 11 à 16, caractérisé en ce que l'on fait réagir un composé entièrement E de formule I.

**18.** Procédé pour la préparation des composés de formule générale

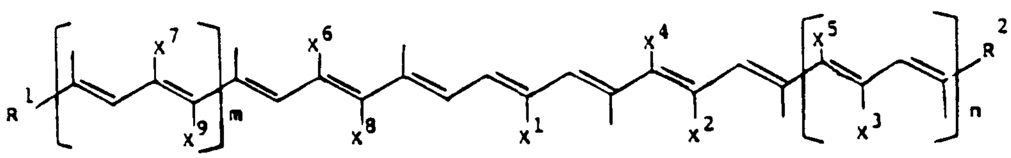

I

où m est égal à 1 et $R^1$ représente le 4-méthyl-3-penténylе ou m est égal à 0 et $R^1$ représente un groupe formyle acétalisé, un groupe carboxy éventuellement estérifié ou un groupe de formule générale

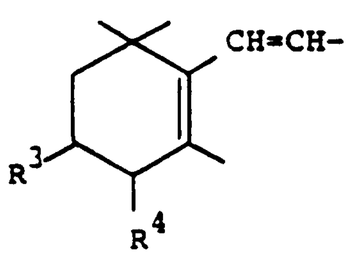

II

n est égal à 1 et $R^2$ représente le 4-méthyl-3-penténylе ou n est égal à 0 et $R^2$ représente un groupe formyle acétalisé, un groupe carboxy éventuellement estérifié ou un groupe de formule II ; $R^3$ et $R^4$ représentent, indépendamment l'un de l'autre, l'hydrogène, un groupe hydroxy éventuellement protégé ou un groupe oxo protégé ; l'un des groupes $X^1$, $X^2$, $X^3$, $X^4$ et $X^9$ présents représentant un reste sulfonyle et les autres groupes l'hydrogène ; et $X^9$, $X^7$, $X^9$ et $X^9$ représentent l'hydrogène ou, lorsque $R^1$ et $R^2$ ont la même signification, également $X^9$ a la signification de $X^2$, $X^7$ a la signification de $X^3$, $X^9$ a la signification de $X^4$ et $X^9$ a la signification de $X^9$,
caractérisé

a) en ce que l'on fait réagir un composé de formule générale

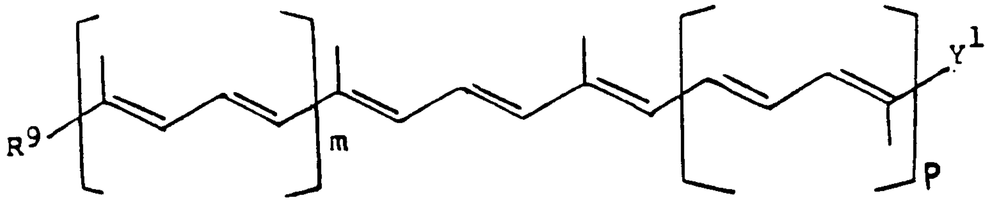

V

avec un composé de formule générale

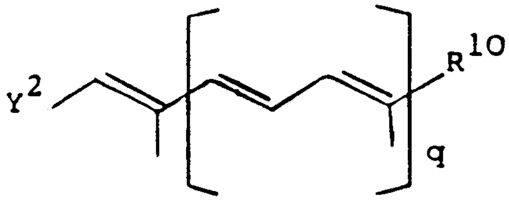

VI

ou

b) en ce que l'on fait réagir un composé de formule générale

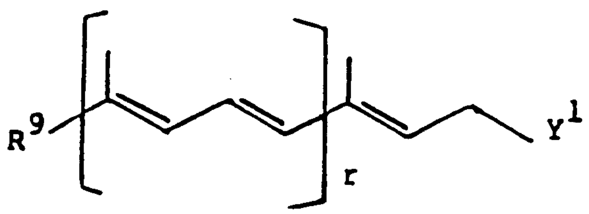

VII

avec un composé de formule générale

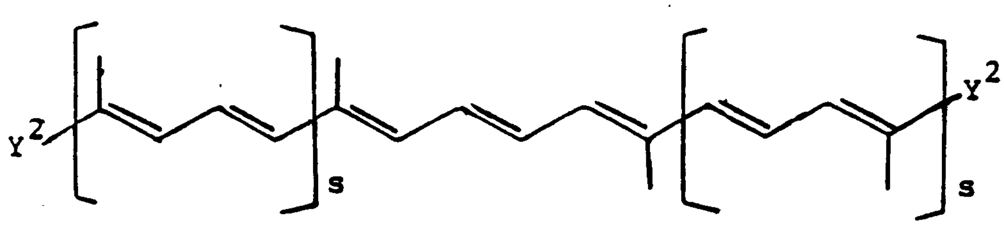

VIII

où l'un des groupes $Y^1$ et $Y^2$ représente le formyle et l'autre groupe représente un groupe $-CH_2X$, X représentant un reste sulfonyle : p et q sont égaux à 0, 1 ou 2 et $p + q = n + 1$ ; r et s sont égaux à 0 ou 1 et $r + s = m$ ; m est égal à 1 et $R^9$ représente le 4-méthyl-3-pentényle, ou m est égal à 0 et $R^9$ représente le groupe formyle acétalisé, un groupe carboxy éventuellement estérifié ou un groupe de formule générale

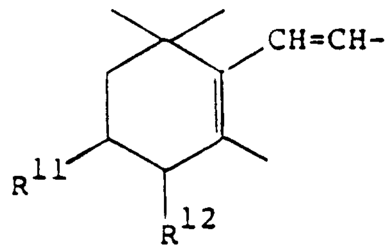

IX

n est égal à 1 et $R^{10}$ représente le 4-méthyl-3-penténylе ou n est égal à 0 et $R^{10}$ représente un groupe formyle acétalisé, un groupe carboxy éventuellement estérifié ou un groupe de formule IX : et $R^{11}$ et $R^{12}$ représentent, indépendamment l'un de l'autre, l'hydrogène, un groupe hydroxy éventuellement protégé ou un groupe oxo protégé,

dans un solvant organique inerte en présence d'une base et en ce que l'on transforme, après addition d'un agent d'acylation, le composé obtenu de formule générale

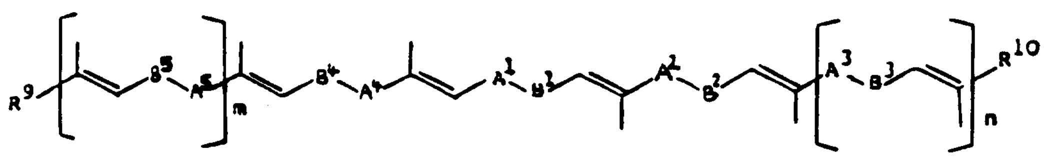

X

où l'un des groupes $-A^1-B^1-$, $-A^2-B^2-$ et $-A^3-B^3-$représente le groupe -CHX-CH(OR)- ou -CH(OR)-CHX- et les deux autres groupes représentant -CH=CH- ; $-A^4-B^4-$ et - $A^5-B^5-$ représentant -CH=CH- ou, lorsque $R^{10}$ a la signification de $R^9$ et n la signification de m, $-A^4-B^4-$ a également la signification de $-A^2-B^2-$ et $-A^5-B^5-$ a également la signification de $-A^3-B^3-$ ; X représente un reste sulfonyle ; -OR représente un acyloxy ; $R^9$, $R^{10}$, m et n ont les significations ci-dessus ; et $R^{11}$ et $R^{12}$ représentent, indépendamment l'un de l'autre, l'hydrogène, un groupe hydroxy protégé ou un groupe oxo protégé, avec une base aqueuse, en un composé de formule I.

**19.** Procédé selon la revendication 18, caractérisé en ce que l'on utilise comme base aqueuse un hydroxyde de métal alcalin ou un hydroxyde de métal alcalino-terreux dans l'eau, de préférence, une lessive de soude ou une lessive de potasse.

**20.** Procédé selon la revendication 18 ou 19, caractérisé en ce que l'on fait réagir les isomères trans ou les isomères entièrement trans des composés de formules V et VI ou des composés de formules VII et VIII.